# EUROPEAN PATENT SPECIFICATION

(11) **EP 3 124 468 B1**
(45) Date of publication and mention of the grant of the patent: **12.12.2018**
(21) Application number: 15770297.8
(22) Date of filing: 23.03.2015
(51) Int. Cl.: C07C 217/76, C09D 5/22, H01L 51/00, H01L 51/50

(54) **FLUORENE DERIVATIVE AND USE THEREOF**
FLUORENDERIVAT UND VERWENDUNG DAVON
DÉRIVÉ DE FLUORÈNE ET SON UTILISATION

(30) Priority: 28.03.2014 JP 2014067494
(43) Date of publication of application: 01.02.2017
(73) Proprietor: Nissan Chemical Industries, Ltd., Tokyo 101-0054 (JP)
(72) Inventor: OTA, Hirofumi, Funabashi-shi Chiba 274-0052 (JP); ENDO, Toshiyuki, Funabashi-shi Chiba 274-0052 (JP)
(74) Representative: Bailey, Sam Rogerson
(86) International application number: PCT/JP2015/058741
(87) International publication number: WO 2015/146912

(56) References cited:
- WO-A1-2010/058777
- WO-A2-2004/090921
- WO-A2-2009/026235
- JP-A- 2007 137 801
- JP-A- 2008 163 306
- JP-A- 2012 188 637
- US-A1- 2007 187 672
- US-A1- 2008 071 049

## Description

### TECHNICAL FIELD

This invention relates to a fluorene derivative and to the use thereof.

### BACKGROUND ART

Charge-transporting thin-films made of organic compounds are used as emissive layers and charge injection layers in organic electroluminescent (EL) devices. In particular, a hole injection layer is responsible for transferring charge between an anode and a hole-transporting layer or an emissive layer, and thus serves an important function in achieving low-voltage driving and high brightness in organic EL devices.

Processes for forming the hole injection layer are broadly divided into dry processes such as vapor deposition and wet processes such as spin coating. Comparing these different processes, wet processes are better able to efficiently produce thin-films having a high flatness over a large area. Hence, with the progress being made today toward larger-area organic EL displays, there exists a desire for hole injection layers that can be formed by wet processes.

In view of these circumstances, the inventors have developed charge-transporting materials which can be employed in various wet processes and which, when used in hole injection layers for organic EL devices, are capable of achieving excellent EL device characteristics. The inventors have also developed compounds of good solubility in organic solvents for use in such charge-transporting materials (see, for example. Patent Documents 1 to 4).

Patent Document 5 discloses charge-transporting varnish comprising a charge-transporting oligoaniline which may comprise a fluorene group.

### PRIOR ART DOCUMENTS

### PATENT DOCUMENTS

Patent Document 1: WO 2008/032616
Patent Document 2: WO 2008/129947
Patent Document 3: WO 2006/025342
Patent Document 4: WO 2010/058777
Patent Document 5: US 2007/0187672

### SUMMARY OF THE INVENTION

### PROBLEMS TO BE SOLVED BY THE INVENTION

It is therefore an object of this invention to provide a fluorene derivative which, as in the art disclosed in the above patent publications, exhibits good solubility in organic solvents and, when formed into a thin-film and used as a hole injection layer, enables an organic EL device endowed with excellent brightness characteristics to be achieved.

### MEANS FOR SOLVING THE PROBLEMS

The inventors have conducted extensive investigations, as a result of which they have discovered that specific fluorene derivatives have an excellent solubility in organic solvents and that thin-films exhibiting high charge transportability can be obtained from varnishes prepared by dissolving such fluorene derivatives in an organic solvent. The inventors have also found that when such a thin-film is used as a hole injection layer in an organic EL device, a device having a high brightness can be obtained.

Accordingly, the invention provides:
1. A fluorene derivative characterized by having formula (1) 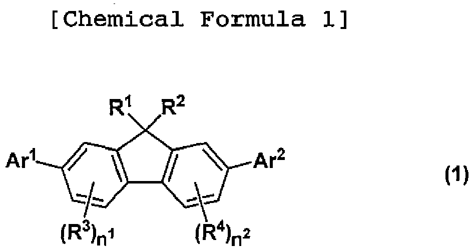
   wherein R¹ and R² are each independently a hydrogen atom, an alkyl group of 1 to 20 carbon atoms, an alkenyl group of 2 to 20 carbon atoms, an alkynyl group of 2 to 20 carbon atoms, an aryl group of 6 to 20 carbon atoms, a heteroaryl group of 2 to 20 carbon atoms, an alkoxy group of 1 to 20 carbon atoms, an alkenyloxy group of 2 to 20 carbon atoms, an alkynyloxy group of 2 to 20 carbon atoms, an aryloxy group of 6 to 20 carbon atoms, a heteroaryloxy group of 2 to 20 carbon atoms, or an alkyl group of 2 to 20 carbon atoms having at least one ether structure (with the proviso that at least one of R¹ and R² is such an alkoxy group, alkenyloxy group, alkynyloxy group, aryloxy group, heteroaryloxy group, or alkyl group having at least one ether structure);
   R³ and R⁴ are each independently a halogen atom, a nitro group, a cyano group, an alkyl group of 1 to 20 carbon atoms which may be substituted with Z¹, an alkenyl group of 2 to 20 carbon atoms which may be substituted with Z¹, an alkynyl group of 2 to 20 carbon atoms which may be substituted with Z¹, an alkoxy group of 1 to 20 carbon atoms which may be substituted with Z¹, an alkenyloxy group of 2 to 20 carbon atoms which may be substituted with Z¹, an alkynyloxy group of 2 to 20 carbon atoms which may be substituted with Z¹, an aryl group of 6 to 20 carbon atoms which may be substituted with Z², a heteroaryl group of 2 to 20 carbon atoms which may be substituted with Z², an aryloxy group of 6 to 20 carbon atoms which may be substituted with Z², or a heteroaryloxy group of 2 to 20 carbon atoms which may be substituted with Z², the respective R³ groups and the respective R⁴ groups being mutually the same or different;
   Z¹ is a halogen atom, a nitro group, a cyano group, an aryl group of 6 to 20 carbon atoms which may be substituted with Z³, a heteroaryl group of 2 to 20 carbon atoms which may be substituted with Z³, an alkoxy group of 1 to 20 carbon atoms which may be substituted with Z³, an alkenyloxy group of 2 to 20 carbon atoms which may be substituted with Z³, an alkynyloxy group of 2 to 20 carbon atoms which may be substituted with Z³, an aryl group of 6 to 20 carbon atoms which may be substituted with Z³, or a heteroaryl group of 2 to 20 carbon atoms which may be substituted with Z³;
   Z² is a halogen atom, a nitro group, a cyano group, an alkyl group of 1 to 20 carbon atoms which may be substituted with Z³, an alkenyl group of 2 to 20 carbon atoms which may be substituted with Z³, an alkynyl group of 2 to 20 carbon atoms which may be substituted with Z³, an alkoxy group of 1 to 20 carbon atoms which may be substituted with Z³, an alkenyloxy group of 2 to 20 carbon atoms which may be substituted with Z³, an alkynyloxy group of 2 to 20 carbon atoms which may be substituted with Z³, an aryl group of 6 to 20 carbon atoms which may be substituted with Z³, or a heteroaryl group of 2 to 20 carbon atoms which may be substituted with Z³;
   Z³ is a halogen atom, a nitro group or a cyano group;
   n¹ and n² represent the number of, respectively, R³ substituents and R⁴ substituents, and are each independently an integer from 0 to 3; and
   Ar¹ and Ar² are each independently a group having any of formulas (A1) to (A13) 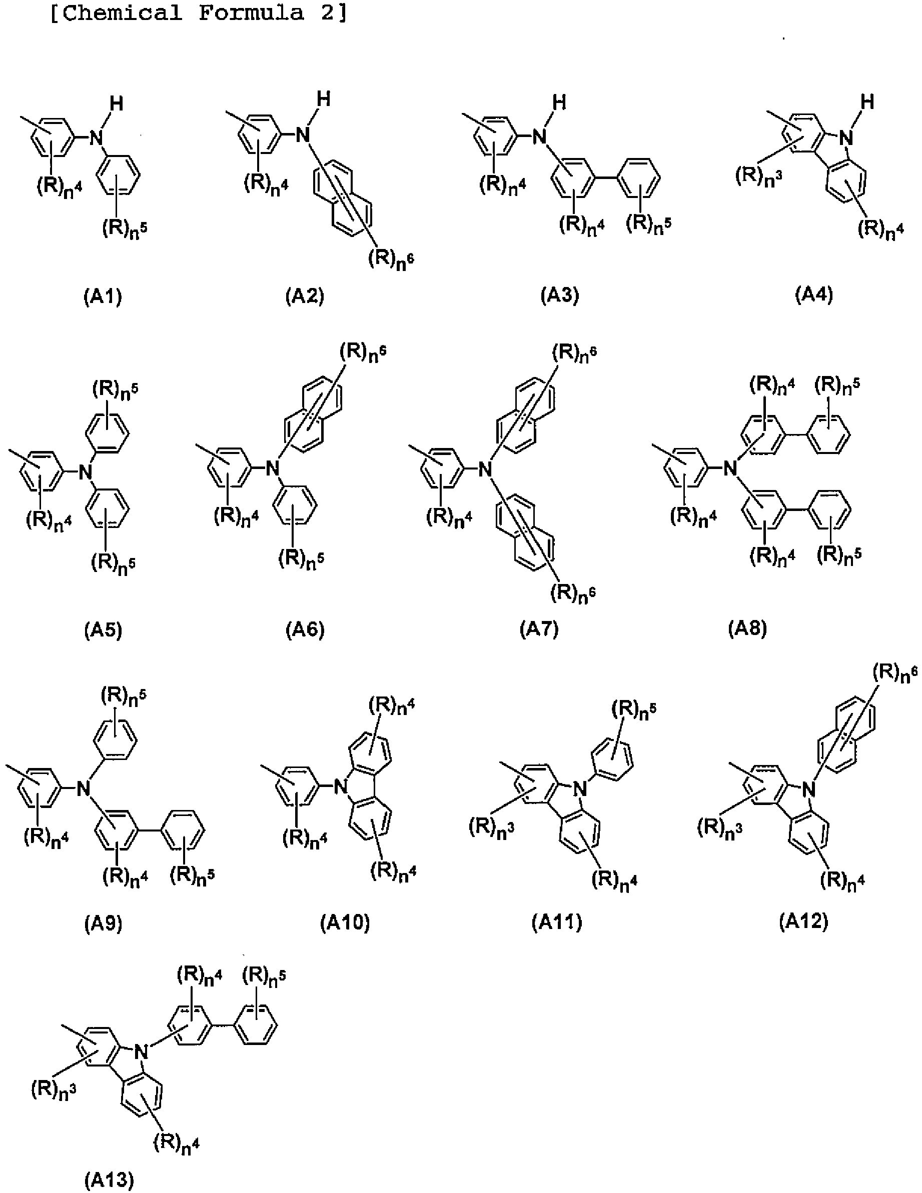 (wherein R is a halogen atom, a nitro group, a cyano group, an alkyl group of 1 to 20 carbon atoms which may be substituted with Z³, an alkenyl group of 2 to 20 carbon atoms which may be substituted with Z³, an alkynyl group of 2 to 20 carbon atoms which may be substituted with Z³, an alkoxy group of 1 to 20 carbon atoms which may be substituted with Z¹, an alkenyloxy group of 2 to 20 carbon atoms which may be substituted with Z³, or an alkynyloxy group of 2 to 20 carbon atoms which may be substituted with Z³, the respective R groups being the same or different; and
   n³ to n⁶ represent the number of R substituents, n³ being an integer from 0 to 3, n⁴ being an integer from 0 to 4, n⁵ being an integer from 0 to 5 and n⁶ being an integer from 0 to 7, with each of n³ to n⁶ being the same or different).
2. The fluorene derivative of 1 above, wherein R¹ and R² are both an alkyl group of 2 to 20 carbon atoms which includes at least one ether structure.
3. The fluorene derivative of 1 or 2 above, wherein n¹ and n² are both 0.
4. A charge-transporting substance consisting of the fluorene derivative of any one of 1 to 3 above.
5. A charge-transporting varnish comprising the charge-transporting substance of 4 above and an organic solvent.
6. The charge-transporting varnish of 5 above which further comprises a dopant substance.
7. A charge-transporting thin-film produced using the charge-transporting varnish of 5 or 6 above.
8. An organic EL device comprising the charge-transporting thin-film of 7 above.
9. A method of preparing the fluorene derivative of 1 above, which method is characterized by comprising the step of carrying out a cross-coupling reaction between a boric acid ester compound of formula (1") or (1"') and compounds of formula (A') and (A") in the presence of a catalyst 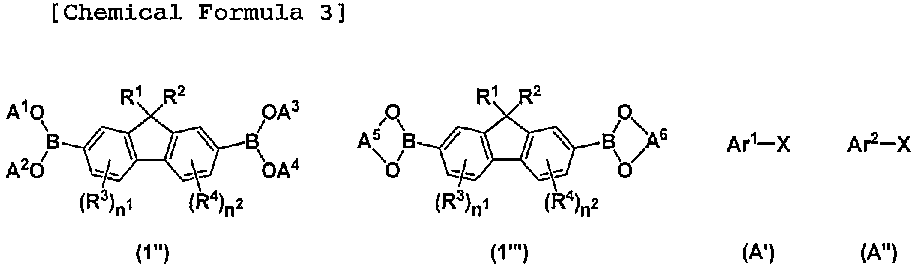 (wherein R¹ to R⁴, Ar¹, Ar², n¹ and n² are as defined above; each X is independently a halogen atom or a pseudo-halogen group; A¹ to A⁴ are each independently a hydrogen atom, an alkyl group of 1 to 20 carbon atoms or an aryl group of 6 to 20 carbon atoms; and A⁵ and A⁶ are each independently an alkanediyl group of 1 to 20 carbon atoms or an arylene group of 6 to 20 carbon atoms).

### ADVANTAGEOUS EFFECTS OF THE INVENTION

Because the fluorene derivative of the invention has excellent solubility in organic solvents, a charge-transporting varnish can easily be prepared by dissolving it in an organic solvent.

A thin-film produced from the charge-transporting varnish of the invention exhibits a high charge-transporting ability, and can thus be advantageously used as a thin-film for organic EL devices and other electronic devices. In particular, because a thin-film obtained from the charge-transporting varnish of the invention has a suitable ionization potential, it can be suitably used as a hole injection layer in an organic EL device.

Also, the charge-transporting varnish of the invention can reproducibly produce thin-films of excellent charge transportability even using various wet processes capable of film formation over a large area, such as spin coating or slit coating, and is thus capable of fully accommodating recent advances in the field of organic EL devices.

### EMBODIMENT FOR CARRYING OUT THE INVENTION

### [Fluorene Derivative]

The fluorene derivative of the invention has formula (1).

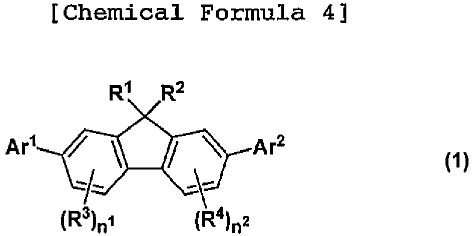

In this formula, R¹ to R⁴ are each independently a hydrogen atom, an alkyl group of 1 to 20 carbon atoms, an alkenyl group of 2 to 20 carbon atoms, an alkynyl group of 2 to 20 carbon atoms, an aryl group of 6 to 20 carbon atoms, a heteroaryl group of 2 to 20 carbon atoms, an alkoxy group of 1 to 20 carbon atoms, an alkenyloxy group of 2 to 20 carbon atoms, an alkynyloxy group of 2 to 20 carbon atoms, an aryloxy group of 6 to 20 carbon atoms, a heteroaryloxy group of 2 to 20 carbon atoms, or an alkyl group of 2 to 20 carbon atoms having at least one ether structure. At least one of R¹ and R² is such an alkoxy group, alkenyloxy group, alkynyloxy group, aryloxy group, heteroaryloxy group, or alkyl group having at least one ether structure.

R³ and R⁴ are each independently a halogen atom, a nitro group, a cyano group, an alkyl group of 1 to 20 carbon atoms which may be substituted with Z¹, an alkenyl group of 2 to 20 carbon atoms which may be substituted with Z¹, an alkynyl group of 2 to 20 carbon atoms which may be substituted with Z¹, an alkoxy group of 1 to 20 carbon atoms which may be substituted with Z¹, an alkenyloxy group of 2 to 20 carbon atoms which may be substituted with Z¹, an alkynyloxy group of 2 to 20 carbon atoms which may be substituted with Z¹, an aryl group of 6 to 20 carbon atoms which may be substituted with Z², a heteroaryl group of 2 to 20 carbon atoms which may be substituted with Z², an aryloxy group of 6 to 20 carbon atoms which may be substituted with Z², or a heteroaryloxy group of 2 to 20 carbon atoms which may be substituted with Z². The respective R³ groups may each be the same or different, and the respective R⁴ groups may each be the same or different.

Z¹ is a halogen atom, a nitro group, a cyano group, an aryl group of 6 to 20 carbon atoms which may be substituted with Z³, a heteroaryl group of 2 to 20 carbon atoms which may be substituted with Z³, an alkoxy group of 1 to 20 carbon atoms which may be substituted with Z³, an alkenyloxy group of 2 to 20 carbon atoms which may be substituted with Z³, an alkynyloxy group of 2 to 20 carbon atoms which may be substituted with Z³, an aryl group of 6 to 20 carbon atoms which may be substituted with Z³, or a heteroaryl group of 2 to 20 carbon atoms which may be substituted with Z³.

Z² is a halogen atom, a nitro group, a cyano group, an alkyl group of 1 to 20 carbon atoms which may be substituted with Z³, an alkenyl group of 2 to 20 carbon atoms which may be substituted with Z³, an alkynyl group of 2 to 20 carbon atoms which may be substituted with Z³, an alkoxy group of 1 to 20 carbon atoms which may be substituted with Z³, an alkenyloxy group of 2 to 20 carbon atoms which may be substituted with Z³, an alkynyloxy group of 2 to 20 carbon atoms which may be substituted with Z³, an aryl group of 6 to 20 carbon atoms which may be substituted with Z³, or an heteroaryl group of 2 to 20 carbon atoms which may be substituted with Z³.

Z³ is a halogen atom, a nitro group or a cyano group.

Specific examples of halogen atoms include fluorine, chlorine, bromine and iodine atoms.

The alkyl group of 1 to 20 carbon atoms may be linear, branched or cyclic, and is exemplified by linear or branched alkyl groups of 1 to 20 carbon atoms such as methyl, ethyl, n-propyl, isopropyl, n-butyl, isobutyl, s-butyl, t-butyl, n-pentyl, n-hexyl, n-heptyl, n-octyl, n-nonyl and n-decyl groups; and cyclic alkyl groups of 3 to 20 carbon atoms such as cyclopropyl, cyclobutyl, cyclopentyl, cyclohexyl, cycloheptyl, cyclooctyl, cyclononyl, cyclodecyl, bicyclobutyl, bicyclopentyl, bicyclohexyl, bicycloheptyl, bicyclooctyl, bicyclononyl and bicyclodecyl groups.

The alkenyl group of 2 to 20 carbon atoms may be linear, branched or cyclic, and is exemplified by ethenyl, n-1-propenyl, n-2-propenyl, 1-methylethenyl, n-1-butenyl, n-2-butenyl, n-3-butenyl, 2-methyl-1-propenyl, 2-methyl-2-propenyl, 1-ethylethenyl, 1-methyl-1-propenyl, 1-methyl-2-propenyl, n-1-pententyl, n-1-decenyl and n-1-eicosenyl groups.

The alkynyl group of 2 to 20 carbon atoms may be linear, branched or cyclic, and is exemplified by ethynyl, n-1-propynyl, n-2-propynyl, n-1-butynyl, n-2-butynyl, n-3-butynyl, 1-methyl-2-propynyl, n-1-pentynyl, n-2-pentynyl, n-3-pentynyl, n-4-pentynyl, 1-methyl-n-butynyl, 2-methyl-n-butynyl, 3-methyl-n-butynyl, 1,1-dimethyl-n-propynyl, n-1-hexynyl, n-1-decynyl, n-1-pentadecynyl and n-1-eicosynyl groups.

Specific examples of aryl groups of 6 to 20 carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl and 9-phenanthryl groups.

Specific examples of heteroaryl groups of 2 to 20 carbon atoms include 2-thienyl, 3-thienyl, 2-furanyl, 3-furanyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl, 2-imidazolyl, 4-imidazolyl, 2-pyridyl, 3-pyridyl and 4-pyridyl groups.

The alkoxy group of 1 to 20 carbon atoms may be linear, branched or cyclic, and is exemplified by linear or branched alkoxy groups of 1 to 20 carbon atoms such as methoxy, ethoxy, n-propoxy, isopropoxy, n-butoxy, isobutoxy, s-butoxy, t-butoxy, n-pentyloxy, n-hexyloxy, n-heptyloxy, n-octyloxy, n-nonyloxy and n-decyloxy groups; and cyclic alkoxy groups of 3 to 20 carbon atoms such as cyclopropyloxy, cyclobutyloxy, cyclopentyloxy, cyclohexyloxy, cycloheptyloxy, cyclooctyloxy, cyclononyloxy, cyclodecyloxy, bicyclobutyloxy, bicyclopentyloxy, bicyclohexyloxy, bicycloheptyloxy, bicyclooctyloxy, bicyclononyloxy and bicyclodecyloxy groups.

The alkenyloxy group of 2 to 20 carbon atoms may be linear, branched or cyclic, and is exemplified by ethenyloxy, n-1-propenyloxy, n-2-propenyloxy, 1-methylethenyloxy, n-1-butenyloxy, n-2-butenyloxy, n-3-butenyloxy, 2-methyl-1-propenyloxy, 2-methyl-2-propenyloxy, 1-ethylethenyloxy, 1-methyl-1-propenyloxy, 1-methyl-2-propenyloxy, n-1-pentenyloxy, n-1-decyloxy and n-1-eicosenyloxy groups.

The alkynyloxy group of 2 to 20 carbon atoms may be linear, branched or cyclic, and is exemplified by ethynyloxy, n-1-propynyloxy, n-2-propynyloxy, n-1-butynyloxy, n-2-butynyloxy, n-3-butynyloxy, 1-methyl-2-propynyloxy, n-1-pentynyloxy, n-2-pentynyloxy, n-3-pentynyloxy, n-4-pentynyloxy, 1-methyl-n-butynyloxy, 2-methyl-n-butynyloxy, 3-methyl-n-butynyloxy, 1,1-dimethyl-n-propynyloxy, n-1-hexynyl, n-1-decynyloxy, n-1-pentadecynyloxy and n-1-eicosynyloxy groups.

The aryloxy group of 6 to 20 carbon atoms is exemplified by phenyloxy, 1-naphthyloxy, 2-naphthyloxy, 1-anthryloxy, 2-anthryloxy, 9-anthryloxy, 1-phenanthryloxy, 2-phenanthryloxy, 3-phenanthryloxy, 4-phenanthryloxy and 9-phenanthryoxy groups.

The heteroaryloxy group of 2 to 20 carbon atoms is exemplified by 2-thienyloxy, 3-thienyloxy, 2-furanyloxy, 3-furanyloxy, 2-oxazolyloxy, 4-oxazolyloxy, 5-oxazolyloxy, 3-isooxazolyloxy, 4-isooxazolyloxy, 5-isooxazolyloxy, 2-thiazolyloxy, 4-thiazolyloxy, 5-thiazolyloxy, 3-isothiazolyloxy, 4-isothiazolyloxy, 5-isothiazolyloxy, 2-imidazolyloxy, 4-imidazolyloxy, 2-pyridyloxy, 3-pyridyloxy and 4-pyridyloxy groups.

The alkyl group of 2 to 20 carbon atoms having at least one ether structure is exemplified by linear or branched alkyl groups in which at least one methylene group is substituted with an oxygen atom, provided it is not one in which a methylene group bonded to the fluorene skeleton is substituted with an oxygen atom and not one in which neighboring methylene groups are at the same time substituted with oxygen atoms. Taking into consideration the availability of the starting compounds, this group is preferably a group of formula (A), and more preferably a group of formula (B).

- (R^{A}O)ᵣ-R⁸ (A)

- (CH₂CH₂O)ᵣ-CH₃ (B)

In these formulas, R^{A} is a linear or branched alkylene group of 1 to 4 carbon atoms, R^{B} is a linear or branched alkyl group having a number of carbon atoms that is from 1 to [20 - (number of carbon atoms in R^{A}) × r], and the subscript r is an integer from 1 to 9. From the standpoint of compatibility with a dopant, r is preferably 2 or more, and more preferably 3 or more. From the standpoint of availability of the starting compounds, r is preferably 5 or less, and more preferably 4 or less.

Illustrative examples of alkyl groups of 2 to 20 carbon atoms which include at least one ether structure include -CH₂OCH₃, -CH₂OCH₂CH₃, -CH₂O(CH₂)₂CH₃, -CH₂OCH(CH₃)₂, -CH₂O(CH₂),CH₃, -CH₂OCH₂CH(CH₃)₂, -CH₂O(CH₃)₃, -CH₂O(CH₂)₄CH₃, -CH₂OCH(CH₃)(CH₂)₂CH₃, -CH₂O(CH₂)₂(CH₃)₂, -CH₂OCH(CH₃)(CH₂)₃CH₃, -CH₂O (CH₂)₅CH₃, -CH₂OCH₂CH(CH₃)(CH₂)₂CH₃, -CH₂O(CH₂)₂CH(CH₃)CH₂CH₃, -CH₂O(CH₂)₃CH(CH₃)₂, -CH₂OC(CH₃)₂(CH₂)₂CH₃, -CH₂OCH(CH₂CH₃)(CH₂)₂CH₃, -CH₂OC(CH₃)₂CH(CH₃)₂, -CH₂O(CH₂)₆CH₃, -CH₂O(CH₂)₇CH₃, -CH₂OCH₂CH(CH₂CH₃)(CH₂)₃CH₃, -CH₂O(CH₂)₈CH₃, -CH₂O(CH₂)₉CH₃, -CH₂O(CH₂)₁₀CH₃, -CH₂O(CH₂)₁₁CH_{3,} -CH₂O(CH₂)₁₂CH₃, -CH₂O(CH₂)₁₃CH₃, -CH₂O(CH₂)₁₄CH_{3,} -CH₂O(CH₂)₁₅CH₃, -CH₂O(CH₂)₁₆CH₃, -CH₂O(CH₂)₁₇CH₃, -CH₂O(CH₂)₁₈CH₃, -CH₂CH₂OCH₃, -CH₂CH₂OCH₂CH₃, -CH₂CH₂O(CH₂)₂CH₃, - CH₂CH₂OCH(CH₃)₂, - CH₂CH₂O(CH₂)₃CH₃, -CH₂CH₂OCH₂CH(CH₃)₂, -CH₂CH₂OC(CH₃)₃, -CH₂CH₂O(CH₂)₄CH₃, -CH₂CH₂OCH(CH₃)(CH₂)₂CH₃, -CH₂CH₂OCH₂CH(CH₃)₂, -CH₂CH₂O(CH₂)₂CH(CH₃)₂, -CH₂CH₂OC(CH₃)₃, -CH₂CH₂OCH(CH₃)(CH₂)₃CH₃, -CH₂CH₂O(CH₂)₅CH₃, - CH₂CH₂OCH(CH₃)(CH₂)₃CH₃, -CH₂CH₂OCH₂CH(CH₃)(CH₂)₂CH₃, - CH₂CH₂O(CH₂)₂CH(CH₃)CH₂CH₃, -CH₂CH₂O(CH₂)₃CH(CH₃)₂, - CH₂CH₂OC(CH₃)₂(CH₂)₂CH₃, -CH₂CH₂OCH(CH₂CH₃)(CH₂)₂CH₃, -CH₂CH₂OC(CH₃)₂CH(CH₃)₂, -CH₂CHO(CH₂)₆CH₃, -CH₂CH₂O(CH₂)₇CH₃, -CH₂CH₂OCH₂CH (CH₂CH₃) (CH₂) ₃CH₃, -CH₂CH₂O(CH₂)₈CH₃, -CH₂CH₂O(CH₂)₉CH₃, -CH₂CH₂O(CH₂)₁₀CH_{3,} -CH₂CH₂O(CH₂)₁₁CH₃, -CH₂CH₂O(CH₂)₁₂CH₃, -CH₂CH₂O(CH₂)₁₃CH₃, -CH₂CH₂O(CH₂)₁₄CH₃, -CH₂CH₂O(CH₂)₁₅CH₃, -CH₂CH₂O(CH₂)₁₆CH₃, -CH₂CH₂O(CH₂)₁₇CH₃, -CH₂CH₂CH₂OCH₃, -CH₂CH₂CH₂OCH₂CH₃, -CH₂CH₂CH₂O(CH₂)₃CH₃, -CH₂CH₂CH₂OCH(CH₃)₂, -CH₂CH₂CH₂O(CH₂)₃CH₃, -CH₂CH₂CH₂OCH₂CH(CH₃)₂, -CH₂CH₂CH₂OC(CH₃)₃, -CH₂CH₂CH₂O(CH₂)₄CH₃, -CH₂CH₂CH₂OCH(CH₃)(CH₂)₂CH₃, -CH₂CH₂CH₂OCH₂CH(CH₃)₂, -CH₂CH₂CH₂O(CH₂)₂CH(CH₃)₂, -CH₂CH₂CH₂OC(CH₃)₃, -CH₂CH₂CH₂OCH(CH₃)(CH₂)₃CH₃, -CH₂CH₂CH₂O(CH₂)₅CH₃, -CH₂CH₂CH₂OCH(CH₃)(CH₂)₃CH₃, - CH₂CH₂CH₂OCH₂CH(CH₃)(CH₂)₂CH₃, -CH₂CH₂CH₂O(CH₂)₂CH(CH₃)CH₂CH₃, - CH₂CH₂CH₂O(CH₂)₃CH(CH₃)₂, -CH₂CH₂CH₂OC(CH₃)₂(CH₂)₂CH₃, - CH₂CH₂CH₂OCH(CH₂CH₃)(CH₂)₂CH₃, -CH₂CH₂CH₂OC(CH₃)₂CH(CH₃)₂, -CH₂CH₂CH₂O(CH₂)₆CH₃, -CH₂CH₂CH₂O(CH₂)₇CH₃, -CH₂CH₂CH₂OCH₂CH(CH₂CH₃)(CH₂)₃CH₃, -CH₂CH₂OCH₂CH₂OCH₃, -CH₂CH₂OCH₂CH₂OCH₂CH₂OCH₃, -CH₂CH₂OCH₂CH₂OCH₂CH₂OCH₂CH₂OCH₃, - CH₂CH₂OCH₂CH₂OCH₂CH₂OCH₂CH₂OCH₂CH₂OCH₃, - CH₂CH₂OCH₂CH₂OCH₂CH₂OCH₂CH₂OCH₂CH₂OCH₂CH₂OCH₃, - CH₂CH₂OCH₂CH₂OCH₂CH₂OCH₂CH₂OCH₂CH₂OCH₂CH₂OCH₂CH₂OCH₃, - CH₂CH₂OCH₂CH₂OCH₂CH₂OCH₂CH₂OCH₂CH₂OCH₂CH₂OCH₂CH₂OCH₂CH₂OCH₃, - CH₂CH₂CH₂OCH₂CH₂CH₂OCH₃, -CH₂CH₂CH₂OCH₂CH₂CH₂OCH₂CH₂CH₂OCH₃, - CH₂CH₂CH₂OCH₂CH₂CH₂OCH₂CH₂CH₂OCH₂CH₂CH₂OCH₃, - CH₂CH₂CH₂OCH₂CH₂CH₂OCH₂CH₂CH₂OCH₂CH₂CH₂OCH₂CH₂CH₂OCH₃, - CH₂CH₂CH₂OCH₂CH₂CH₂OCH₂CH₂CH₂OCH₂CH₂CH₂OCH₂CH₂CH₂OCH₂CH₂CH₂OCH₃, - CH₂CH₂CH₂CH₂OCH₂CH₂CH₂CH₂OCH₂CH₂CH₂CH₂OCH₃, - CH₂CH₂CH₂CH₂OCH₂CH₂CH₂CH₂OCH₂CH₂CH₂CH₂OCH₂CH₂CH₂CH₂OCH₃, - CH₂CH₂OCH₂CH₂OCH₂CH₂OCH₂CH₂OCH₂CH₂OCH₂CH₂OCH₂CH₃, - CH₂CH₂OCH₂CH₂OCH₂CH₂OCH₂CH₂OCH₂CH₂OCH₂CH₂OCH₂CH₂OCH₂CH₃, - CH₂CH₂OCH₂CH₂OCH₂CH₂OCH₂CH₂OCH₂CH₂OCH₂CH₂OCH₂CH₂OCH₂CH₂OCH₂CH₃, -CH₂CH₂OCH₂CH₂OCH₂CH₃, -CH₂CH₂OCH₂CH₂OCH₂CH₂OCH₂CH₃, -CH₂CH₂CH₂O(CH₂)₈CH₃, -CH₂CH₂CH₂O(CH₂)₉CH₃, -CH₂CH₂CH₂O(CH₂)₁₀CH₃, -CH₂CH₂CH₂O(CH₂)₁₁CH₃, -CH₂CH₂CH₂O(CH₂)₁₂CH₃, -CH₂CH₂CH₂O(CH₂)₁₃CH₃, -CH₂CH₂CH₂O(CH₂)₁₄CH₃, -CH₂CH₂CH₂O(CH₂)₁₅CH₃ and -CH₂CH₂CH₂O(CH₂)₁₆CH₃**.**

At least one of R¹ and R² is such an alkoxy group, alkenyloxy group, alkynyloxy group, aryloxy group, heteroaryloxy group, or alkyl group having at least one ether structure, with preferably both being any of these groups.

From the standpoint of ease of synthesis, R¹ and R² are preferably the same group.

In formula (1), n¹ and n² represent the number of, respectively, R³ substituents and R⁴ substituents, and are each independently an integer from 0 to 3. From the standpoint of enhancing the charge transportability of the fluorene derivative of the invention, they are preferably from 0 to 2, more preferably 0 or 1, and most preferably 0. It is especially preferable for n¹ and n² to both be 0.

In formula (1), Ar¹ and Ar² are each independently a group having any of formulas (A1) to (A13).

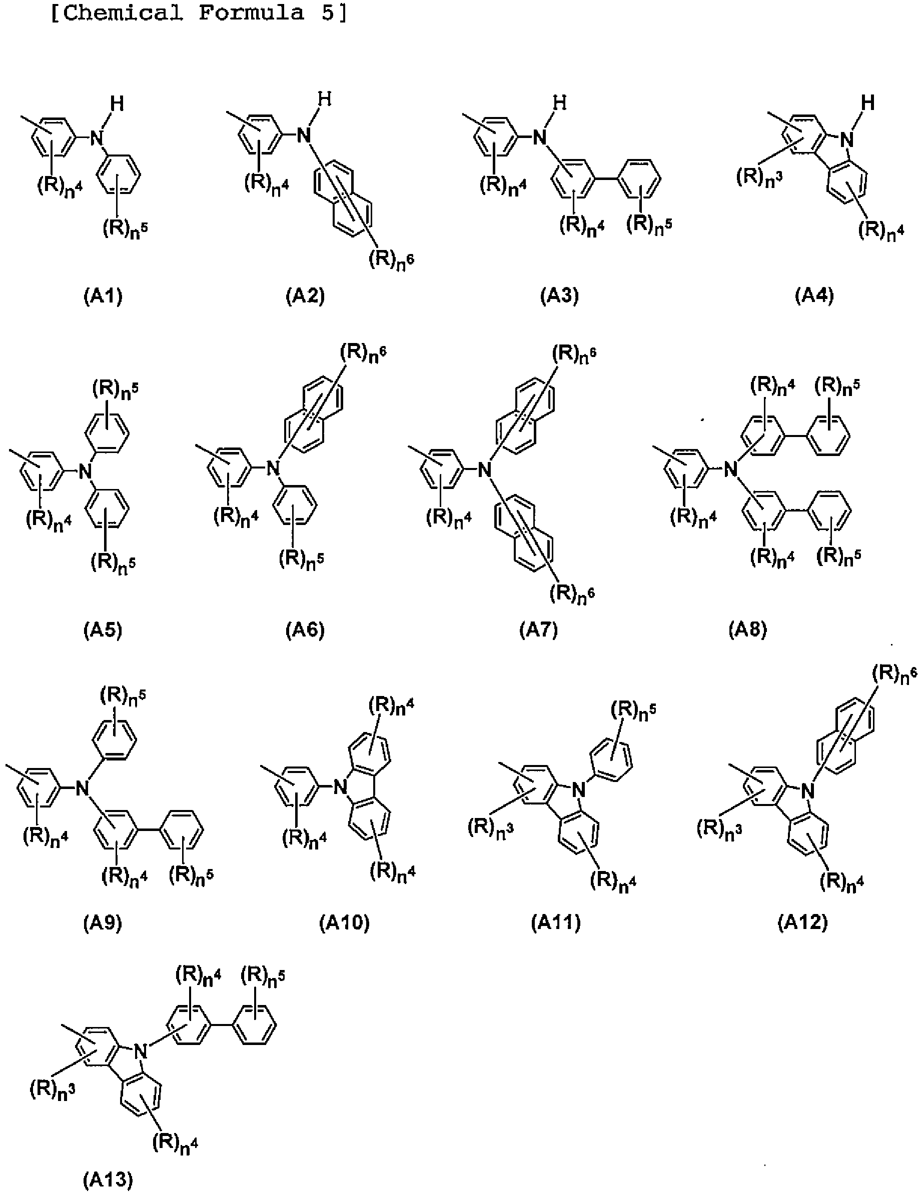

Of these, a group of any of formulas (A1') to (A13') is preferred.

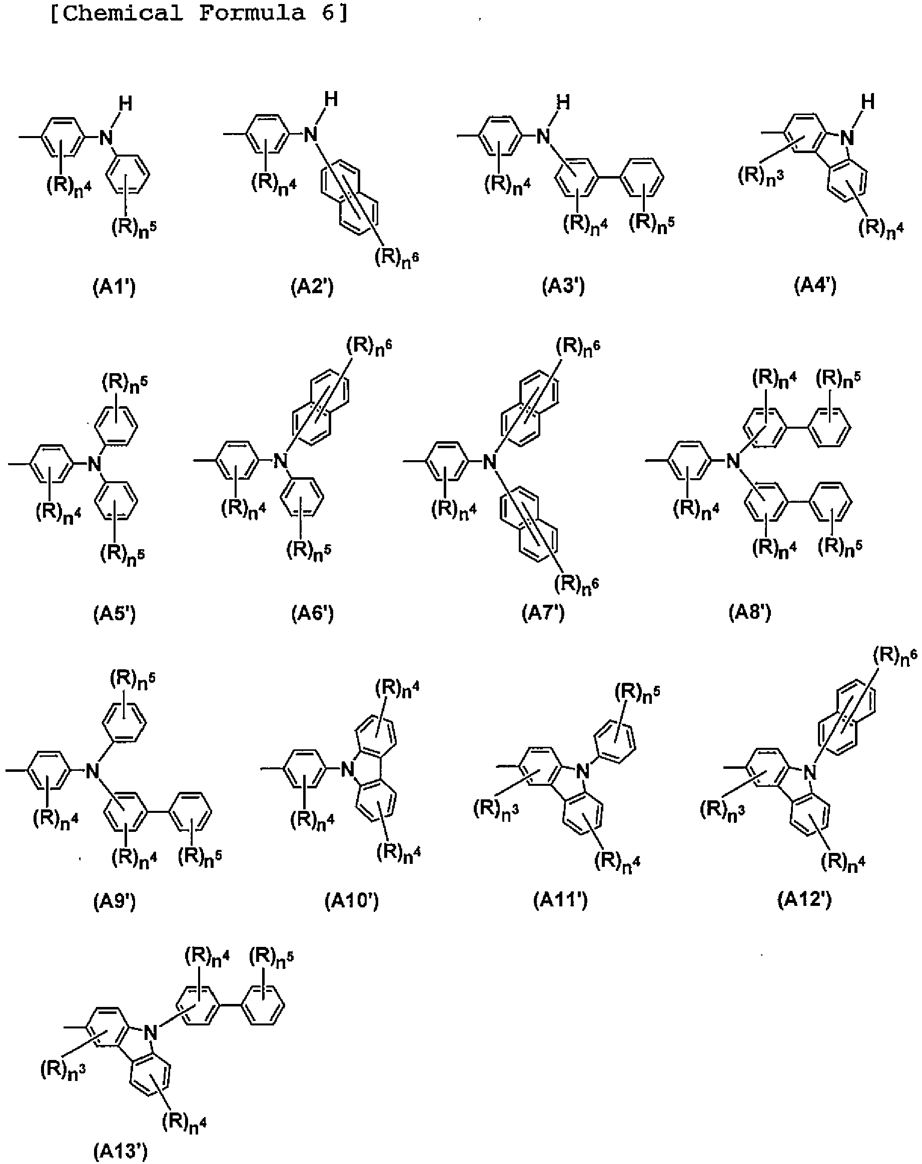

In these formulas, R is a halogen atom, a nitro group, a cyano group, an alkyl group of 1 to 20 carbon atoms which may be substituted with Z³, an alkenyl group of 2 to 20 carbon atoms which may be substituted with Z³, an alkynyl group of 2 to 20 carbon atoms which may be substituted with Z³, an alkoxy group of 1 to 20 carbon atoms which may be substituted with Z¹, an alkenyloxy group of 2 to 20 carbon atoms which may be substituted with Z³, or an alkynyloxy group of 2 to 20 carbon atoms which may be substituted with Z³. Each R may be the same or may be different.

These alkyl groups, alkenyl groups, alkynyl groups, alkoxy groups, alkenyloxy groups and alkynyloxy groups are exemplified in the same way as above.

In the formulas, n³ to n⁶ represent the number of R substituents, n³ being an integer from 0 to 3, n⁴ being an integer from 0 to 4, n⁵ being an integer from 0 to 5 and n⁶ being an integer from 0 to 7. From the standpoint of charge transportability, n³ to n⁶ are each preferably from 0 to 2, more preferably 0 or 1, and most preferably 0. The subscripts n³ to n⁶ may be mutually the same or different.

Of the above, from the standpoint of improving the solubility of the fluorene derivative of the invention in organic solvents, groups of formulas (A1) and (A5) are preferred, and groups of formulas (A1') and (A5') are more preferred. From the standpoint of deepening the ionization potential of a thin-film obtained from a varnish containing the fluorene derivative of the invention and an organic solvent, groups of formulas (A5) to (A13) are preferred, and groups of formulas (A5') to (13') are more preferred.

Ar¹ and Ar² are preferably at the same time any groups of formulas (A1) to (A13), and are more preferably at the same time any groups of formulas (A1') to (A13').

### [Method of Synthesizing Fluorene Derivative]

The fluorene derivative of the invention can be synthesized using, for example, the Suzuki-Miyaura coupling reaction after first synthesizing an intermediate of formula (1') in accordance with Scheme A below. 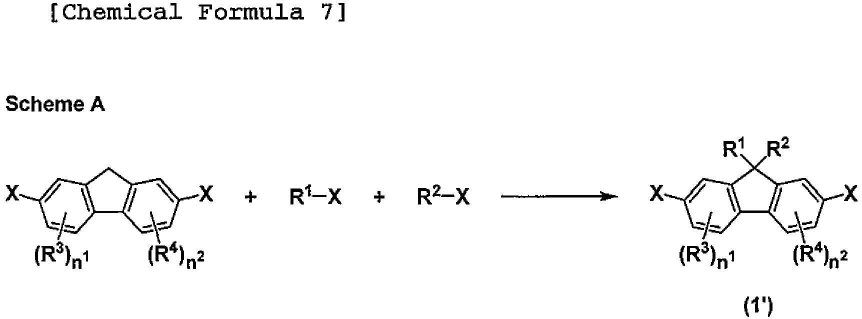 Here, R¹ to R⁴, n¹ and n² are as defined above. Each X is independently a halogen atom or a pseudo-halogen group.

The halogen atom is exemplified by fluorine, chlorine, bromine and iodine atoms. The pseudo-halogen group is exemplified by fluoroalkylsulfonyloxy groups such as methanesulfonyloxy, trifluoromethanesulfonyloxy and nanofluorobutanesulfonyloxy groups; and aromatic sulfonyloxy groups such as benzenesulfonyloxy and toluenesulfonyloxy groups.

An example of a method of synthesizing the fluorene derivative of the invention from an intermediate of formula (1') using the Suzuki-Miyaura coupling reaction is shown below.

First, as shown in Scheme B1 below, a boric acid ester compound (1") is synthesized by reacting an intermediate of formula (1') with a diboric acid ester of formula (B) in the presence of a catalyst. 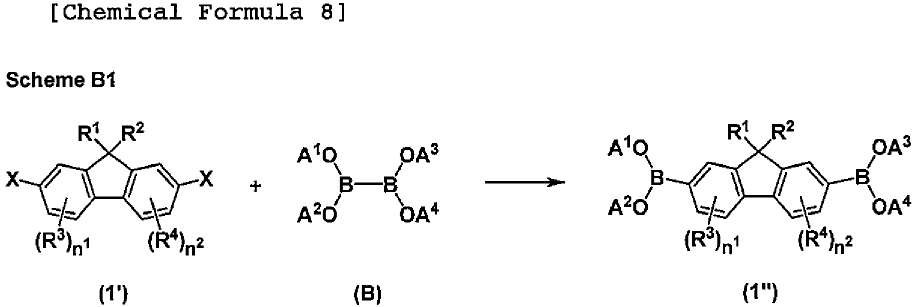 Here, R¹ to R⁴, X, n¹ and n² are as defined above. A¹ to A⁴ are each independently a hydrogen atom, an alkyl group of 1 to 20 carbon atoms or an aryl group of 6 to 20 carbon atoms.

Alternatively, as shown in Scheme B2 below, a boric acid ester compound (1"') is synthesized by reacting an intermediate of formula (1') with a boric acid ester of formula (B') in the presence of a catalyst. 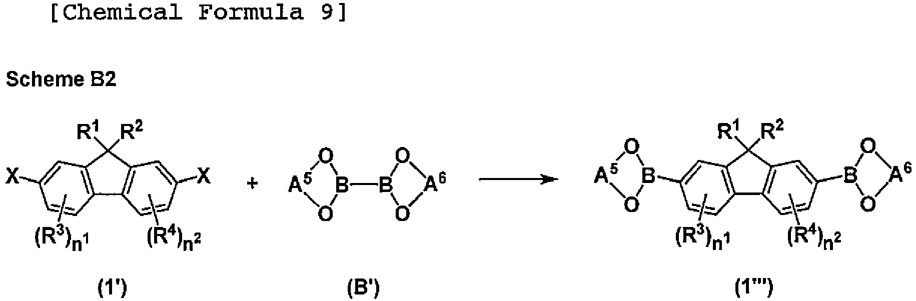 Here, R¹ to R⁴, X, n¹ and n² are as defined above. A⁵ to A⁶ are each independently an alkanediyl group of 1 to 20 carbon atoms or an arylene group of 6 to 20 carbon atoms.

Such alkyl groups or aryl groups are exemplified in the same way as above.

Illustrative examples of the alkanediyl group of 1 to 20 carbon atoms include methylene, ethylene, propan-1,2-diyl, propan-1,3-diyl, 2,2-dimethylpropan-1,3-diyl, 2-ethyl-2-methylpropan-1,3-diyl, 2,2-diethylpropan-1,3-diyl, 2-methyl-2-propylpropan-1,3-diyl, butan-1,3-diyl, butan-2,3-diyl, butan-1,4-diyl, 2-methylbutan-2,3-diyl, 2,3-dimethylbutan-2,3-diyl, pentan-1,3-diyl, pentan-1,5-diyl, pentan-2,3-diyl, pentan-2,4-diyl, 2-methylpentan-2,3-diyl, 3-methylpentan-2,3-diyl, 4-methylpentan-2,3-diyl, 2,3-dimethylpentan-2,3-diyl, 3-methylpentan-2,4-diyl, 3-ethylpentan-2,4-diyl, 3,3-dimethylpentan-2,4-diyl, 3,3-dimethylpentan-2,4-diyl, 2,4-dimethylpentan-2,4-diyl, hexan-1,6-diyl, hexan-1,2-diyl, hexan-1,3-diyl, hexan-2,3-diyl, hexan-2,4-diyl, hexan-2,5-diyl, 2-methylhexan-2,3-diyl, 4-methylhexan-2,3-diyl, 3-methylhexan-2,4-diyl, 2,3-dimethylhexan-2,4-diyl, 2,4-dimethylhexan-2,4-diyl, 2,5-dimethylhexan-2,4-diyl, 2-methylhexan-2,5-diyl, 3-methylhexan-2,5-diyl and 2,5-dimethylhexan-2,5-diyl groups.

Illustrative examples of arylene groups of 6 to 20 carbon atoms include 1,2-phenylene, 1,2-naphthylene, 2,3-naphthylene, 1,8-naphthylene, 1,2-anthrylene, 2,3-anthrylene, 1,2-phenanthrylene, 3,4-phenanthrylene and 9,10-phenanthrylene groups.

The catalyst used in the reaction in Scheme B1 or B2 is exemplified by palladium catalysts such as [1,1'-bis(diphenylphosphino)ferrocene]palladium(II) dichloride (PdCl₂(dppf)), tetrakis(triphenylphosphine)palladium (Pd(PPh₃)₄), bis(triphenylphosphine)dichloropalladium (Pd(PPh₃)₂Cl₂), bis(benzylideneacetone)palladium (Pd(dba)₂), tris(benzylideneacetone)dipalladium (Pd₂(dba)₃) and bis(tri-t-butylphosphine)palladium (Pd(P-t-Bu₃)₂).

Next, as shown in Scheme C1 or C2 below, the fluorene derivative of formula (1) can be synthesized by using boric acid ester compound (1") or (1"') and the compounds of formula (A') and (A") to carry out a cross-coupling reaction in the presence of a catalyst. 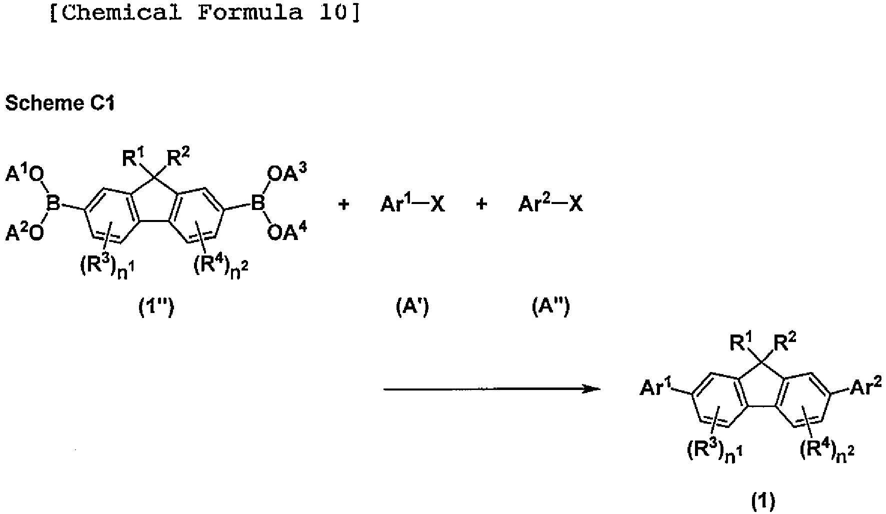 Here, R¹ to R⁴, Ar¹, Ar², X, A¹ to A⁴, n¹ and n² are as defined above. 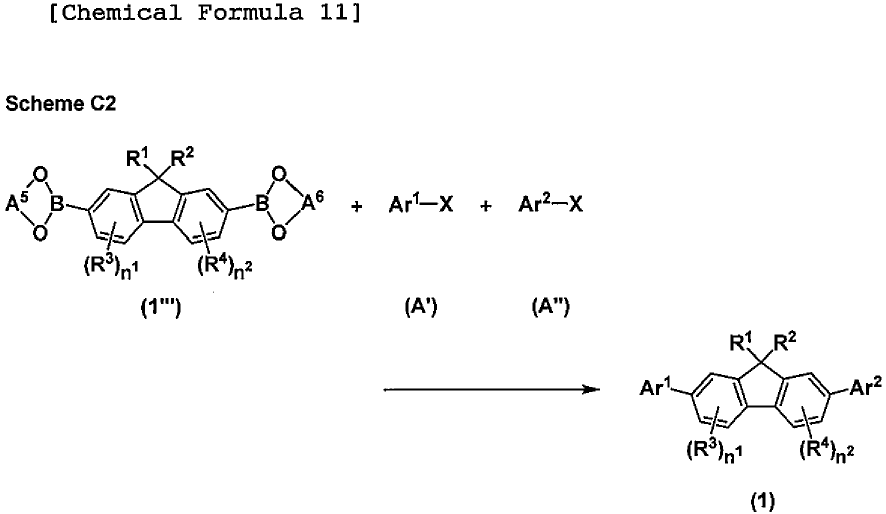 Here, R¹ to R⁴, Ar¹, Ar², X, A⁵, A⁶, n¹ and n² are as defined above.

The catalyst used in the reaction of Scheme C1 or C2 is exemplified by the above-mentioned palladium catalysts.

The solvent used in the reaction of Scheme B1 or B2 and the reaction of Scheme C1 or C2 is preferably an aprotic polar organic solvent, examples of which include N,N-dimethylformamide, N,N-dimethylacetamide, N-methylpyrrolidone, 1,3-dimethyl-2-imidazolidinone, dimethylsulfoxide, tetrahydrofuran and dioxane. From the standpoint of the ease of removing the reaction solvent following the reaction, N,N-dimethylformamide, N,N-dimethylacetamide, tetrahydrofuran, dioxane and the like are preferred.

The reaction temperature may be generally from -50°C up to the boiling point of the solvent used, although the range of 0 to 140°C is preferred. The reaction time is generally from 0.1 to 100 hours.

Following reaction completion, the target fluorene derivative can be obtained by work-up in the usual manner.

### [Charge-Transporting Varnish]

The charge-transporting varnish of the invention includes a charge-transporting substance consisting of the fluorene derivative, and an organic solvent.

### [Organic Solvent]

Highly solvating solvents which are capable of dissolving well the charge-transporting substance and the subsequently described dopant may be used as the organic solvent employed when preparing the charge-transporting varnish.

Examples of such highly solvating solvents that may be used include, but are not limited to, organic solvents such as cyclohexanone, N,N-dimethylformamide, N,N-dimethylacetamide, N-methylpyrrolidone and 1,3-dimethyl-2-imidazolidinone. These solvents may be used singly, or two or more may be used in admixture. The amount thereof may be set to from 5 to 100 wt%, based on the overall solvent used in the varnish.

The charge-transporting substance and dopant are preferably in a state where both are either completely dissolved or uniformly dispersed in the solvent, and are more preferably completely dissolved.

In the invention, at least one high-viscosity organic solvent having a viscosity at 25°C of 10 to 200 mPa·s, especially 35 to 150 mPa·s, and a boiling point at standard pressure (atmospheric pressure) of 50 to 300°C, especially 150 to 250°C may be included in the varnish. By adding such a solvent, the viscosity of the varnish is easily adjusted, making it possible to prepare a varnish which reproducibly gives thin-films of high flatness and is suitable for the coating method to be used.

Examples of the high-viscosity organic solvent include, but are not limited to, cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol and hexylene glycol.

The amount of high-viscosity organic solvent added as a proportion of the overall solvent used in the varnish of the invention is preferably within a range where no precipitation of solids occurs. The amount of such addition is preferably 5 to 80 wt%, provided that no precipitation of solids occurs.

In addition, other solvents may be admixed in a proportion with respect to the overall solvent used in the varnish of 1 to 90 wt%, and preferably 1 to 50 wt%, for such purposes as to enhance the substrate wettability by the varnish, adjust the solvent surface tension, adjust the polarity, and adjust the boiling point.

Examples of such solvents include, but are not limited to, propylene glycol monomethyl ether, ethylene glycol monobutyl ether, diethylene glycol diethyl ether, diethylene glycol monomethyl ether, diethylene glycol dimethyl ether, diethylene glycol monoethyl ether acetate, diethylene glycol monobutyl ether acetate, dipropylene glycol monomethyl ether, propylene glycol monomethyl ether acetate, diethylene glycol monoethyl ether, diacetone alcohol, γ-butyrolactone, ethyl lactate and n-hexyl acetate. These solvents may be used singly, or two or more may be used in admixture.

The viscosity of the inventive varnish is set as appropriate for the thickness and other properties of the thin-film to be produced and the solids concentration of the varnish, but is generally from 1 to 50 mPa·s at 25°C. Also, the solids concentration of the charge-transporting varnish of the invention is set as appropriate based on such considerations as the viscosity, surface tension and other properties of the varnish and the thickness and other properties of the thin-film to be produced, and is generally from about 0.1 to about 10.0 wt%. To improve the coating properties of the varnish, the solids concentration of the varnish is preferably from about 0.5 to about 5.0 wt%, and more preferably from about 1.0 to about 3.0 wt%. Here, "solids" refers to the varnish components that remain after the organic solvent has been removed.

### [Dopant]

Depending on the intended use of the thin-film to be obtained, the charge-transporting varnish of the invention may include a dopant for the purpose of, for example, enhancing the charge transportability. The dopant is not particularly limited, provided it dissolves in at least one of the solvents used in the varnish. The dopant may be either an inorganic dopant or an organic dopant.

When a dopant is used, the included amount thereof varies according to the type of dopant and cannot be strictly specified, but is generally from about 0.5 to about 5.0 parts by weight per part by weight of the fluorene derivative of the invention.

Examples of inorganic dopants include inorganic acids such as hydrogen chloride, sulfuric acid, nitric acid and phosphoric acid; metal halides such as aluminum(III) chloride (AlCl₃), titanium(IV) tetrachloride (TiCl₄), boron tribromide (BBr₃), a boron trifluoride-ether complex (BF₃·OEt₂), iron(III) chloride (FeCl₃), copper(II) chloride (CuCl₂), antimony(V) pentachloride (SbCl₅), antimony(V) pentafluoride (SbF₅), arsenic(V) pentafluoride (AsF₅), phosphorus pentafluoride (PF₅) and tris(4-bromophenyl)aluminum hexachloroantimonate (TBPAH); halogens such as Cl₂, Br₂, I₂, ICl, ICl₃, IBr and IF₄; and heteropolyacids such as phosphomolybdic acid and phosphotungstic acid.

Examples of organic dopants include arylsulfone compounds such as benzenesulfonic acid, tosylic acid, p-styrenesulfonic acid, 2-naphthalenesulfonic acid, 4-hydroxybenzenesulfonic acid, 5-sulfosalicyclic acid, p-dodecylbenzenesulfonic acid, dihexylbenzenesulfonic acid, 2,5-dihexylbenzenesulfonic acid, dibutylnaphthalenesulfonic acid, 6,7-dibutyl-2-naphthalenesulfonic acid, dodecylnaphthalenesulfonic acid, 3-dodecyl-2-naphthalenesulfonic acid, hexylnaphthalenesulfonic acid, 4-hexyl-1-naphthalenesulfonic acid, octylnaphthalenesulfonic acid, 2-octyl-1-naphthalenesulfonic acid, hexylnaphthalenesulfonic acid, 7-hexyl-1-naphthalenesulfonic acid, 6-hexyl-2-naphthalenesulfonic acid, dinonylnaphthalenesulfonic acid, 2,7-dinonyl-4-naphthalenesulfonic acid, dinonylnaphthalenedisulfonic acid, 2,7-dinonyl-4,5-naphthalenedisulfonic acid, the 1,4-benzodioxanedisulfonic acid compounds mentioned in WO 2005/000832,
the arylsulfonic acid compounds mentioned in WO 2006/025342, the arylsulfonic acid compounds mentioned in WO 2009/096352, and polystyrenesulfonic acids.
These inorganic and organic dopants may be used singly or two or more may be used in combination.

Preferred dopants include, but are not limited to, heteropolyacids such as phosphotungstic acid, and arylsulfonic acid compounds of the following formulas.

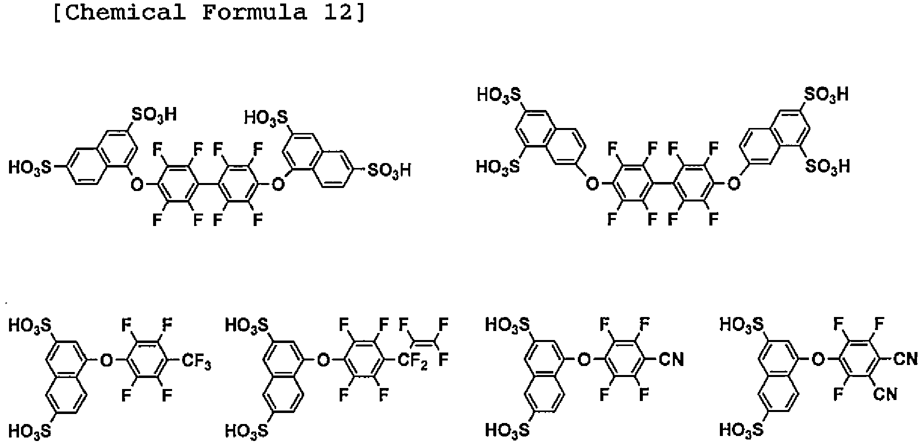

### [Method of Preparing Charge-Transporting Varnish]

Examples of methods for preparing the charge-transporting varnish include, but are not particularly limited to, the approach of dissolving the charge-transporting substance, dopant, etc. in a highly solvating solvent and then adding thereto a high-viscosity organic solvent, and the approach of mixing together a highly solvating solvent and a high-viscosity organic solvent and then dissolving therein the charge-transporting substance of the invention, dopant, etc.

In this invention, from the standpoint of reproducibly obtaining a higher flatness thin-film, it is desirable for the charge-transporting varnish to be obtained by dissolving the charge-transporting substance, dopant, etc. in the organic solvent, then filtering the solution using a submicron-order filter or the like.

### [Charge-Transporting Thin-Film]

A charge-transporting thin-film can be formed on a substrate by coating the charge-transporting varnish of the invention onto the substrate and baking.

Examples of the varnish coating method include, but are not particularly limited to, dipping, spin coating, transfer printing, roll coating, brush coating, inkjet printing, spraying and slit coating. The viscosity and surface tension of the varnish are preferably adjusted according to the coating method to be used.

When using the varnish of the invention, the baking atmosphere is not particularly limited. A thin-film having a uniform film surface and high charge transportability can be obtained not only in an open-air atmosphere, but even in an inert gas such as nitrogen or in a vacuum. However, to more reproducibly obtain a thin-film having an excellent charge transportability, an open-air atmosphere is preferred.

The baking temperature is suitably set in the range of about 100 to 260°C while taking into account such factors as the intended use of the resulting thin-film and the degree of charge transportability to be imparted to the thin-film. When the thin-film thus obtained is to be used as a hole injection layer in an organic EL device, the baking temperature is preferably about 140 to 250°C, and more preferably about 145 to 240°C.

During baking, a temperature change in two or more steps may be applied for such purposes as to achieve more uniform film formability or to induce the reaction to proceed on the substrate. Heating may be carried out using a suitable apparatus such as a hot plate or an oven.

The thickness of the charge-transporting thin-film is not particularly limited. However, when the thin-film is to be used as a hole injection layer in an organic EL device, a film thickness of from 5 to 200 nm is preferred. Methods for changing the film thickness include, for example, changing the solids concentration in the varnish and changing the amount of solution on the substrate during coating.

### [Organic EL Device]

The organic EL device of the invention has a pair of electrodes and, between these electrodes, the above-described charge-transporting thin-film of the invention.

Typical organic EL device configurations include, but are not limited to, those of (a) to (f) below. In these configurations, where necessary, an electron-blocking layer or the like may be provided between the emissive layer and the anode, and a hole-blocking layer or the like may be provided between the emissive layer and the cathode. Alternatively, the hole injection layer, hole-transporting layer or hole injecting and transporting layer may also have the function of, for example, an electron-blocking layer; and the electron injection layer, electron-transporting layer or electron injecting and transporting layer may also have the function of, for example, a hole-blocking layer.
(a) anode/hole injection layer/hole-transporting layer/emissive layer/electron-transporting layer/electron injection layer/cathode
(b) anode/hole injection layer/hole-transporting layer/emissive layer/electron injecting and transporting layer/cathode
(c) anode/hole injecting and transporting layer/emissive layer/electron-transporting layer/electron injection layer/cathode
(d) anode/hole injecting and transporting layer/emissive layer/electron injecting and transporting layer/cathode
(e) anode/hole injection layer/hole-transporting layer/emissive layer/cathode
(f) anode/hole injecting and transporting layer/emissive layer/cathode

As used herein, "hole injection layer," "hole-transporting layer" and "hole injecting and transporting layer" refer to layers which are formed between the emissive layer and the anode, and which have the function of transporting holes from the anode to the emissive layer. When only one layer of hole-transporting material is provided between the emissive layer and the anode, this is a "hole injecting and transporting layer"; when two or more layers of hole-transporting material are provided between the emissive layer and the anode, the layer that is closer to the anode is a "hole injection layer" and the other layer is a "hole-transporting layer." In particular, thin-films having not only an ability to receive holes from the anode but also an excellent ability to inject holes into, respectively, the hole-transporting layer and the emissive layer may be used as the hole injection layer and the hole injecting and transporting layer.

In addition, "electron injection layer," "electron-transporting layer" and "electron injecting and transporting layer" refer to layers which are formed between the emissive layer and the cathode, and which have the function of transporting electrons from the cathode to the emissive layer. When only one layer of electron-transporting material is provided between the emissive layer and the cathode, this is an "electron injecting and transporting layer"; when two or more layers of electron-transporting material are provided between the emissive layer and the cathode, the layer that is closer to the cathode is an "electron injection layer" and the other layer is an "electron-transporting layer."

The "emissive layer" is an organic layer having a light-emitting function. When a doping system is used, this layer includes a host material and a dopant material. The function of the host material is primarily to promote the recombination of electrons and holes, and to confine the resulting excitons within the emissive layer. The function of the dopant material is to cause the excitons obtained by recombination to efficiently luminesce. In the case of a phosphorescent device, the host material functions primarily to confine within the emissive layer the excitons generated by the dopant.

The charge-transporting thin-film of the invention can preferably be used as a hole injection layer, a hole-transporting layer or a hole transporting and injecting layer, and can more preferably can be used as a hole injection layer, in an organic EL device.

The materials and method employed to fabricate an organic EL device using the charge-transporting varnish of the invention are exemplified by, but not limited to, those described below.

The electrode substrate to be used is preferably cleaned beforehand by liquid washing with, for example, a cleaning agent, alcohol or pure water. When the substrate is an anode substrate, it is preferably subjected to surface treatment such as UV/ozone treatment or oxygen-plasma treatment just prior to use. However, surface treatment need not be carried out if the anode material is composed primarily of organic substances.

An example of a method of fabricating an inventive organic EL device in which a thin-film obtained from the charge-transporting varnish of the invention serves as a hole injection layer is described below.

In the method described above, a hole injection layer is formed on an electrode by coating the charge-transporting varnish of the invention onto an anode substrate and baking. A hole-transporting layer, emissive layer, electron-transporting layer, electron injection layer and cathode are then provided in this order on the hole injection layer. The hole-transporting layer, emissive layer, electron-transporting layer and electron injection layer may be formed by vapor deposition processes or coating processes (wet processes), depending on the properties of the material to be used.

Illustrative examples of anode materials include transparent electrodes such as indium-tin oxide (ITO) and indium-zinc oxide (IZO), and metal anodes made of a metal such as aluminum or an alloy of such a metal. An anode material on which planarizing treatment has been carried out is preferred. Use can also be made of polythiophene derivatives and polyaniline derivatives having a high charge transporting ability.

Examples of other metals making up the metal anode include, but are not limited to, scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, bismuth, and alloys thereof.

Specific examples of hole-transporting layer-forming materials include the following hole-transporting low-molecular-weight materials: triarylamines such as (triphenylamine) dimer derivatives, [(triphenylamine) dimer] spirodimer, N,N'-bis(naphthalen-l-yl)-N,N'-bis(phenyl)benzidine (α-NPD), N,N'-bis(naphthalen-2-yl)-N,N'-bis(phenyl)benzidine, N,N'-bis(3-methylphenyl)-N,N'-bis(phenyl)benzidine, N,N'-bis(3-methylphenyl)-N,N'-bis(phenyl)-9,9-spirobifluorene, N,N'-bis(naphthalen-1-yl)-N,N'-bis(phenyl)-9,9-spirobifluorene, N,N'-bis(3-methylphenyl)-N,N'-bis(phenyl)-9,9-dimethylfluorene, N,N'-bis(naphthalen-1-yl)-N,N'-bis(phenyl)-9,9-dimethylfluorene, N,N'-bis(3-methylphenyl)-N,N'-bis(phenyl)-9,9-diphenylfluorene, N,N'-bis(naphthalen-1-yl)-N,N'-bis(phenyl)-9,9-diphenylfluorene, N,N'-bis(naphthalen-l-yl)-N,N'-bis(phenyl)-2,2'-dimethylbenzidine, 2,2',7,7'-tetrakis(N,N-diphenylamino)-9,9-spirobifluorene, 9,9-bis[4-(N,N-bisbiphenyl-4-ylamino)phenyl]-9H-fluorene, 9,9-bis[4-(N,N-bisnaphthalen-2-ylamino)phenyl]-9H-fluorene, 9,9-bis[4-(N-naphthalen-1-yl-N-phenylamino)phenyl]-9H-fluorene, 2,2',7,7'-tetrakis[N-naphthalenyl(phenyl)amino]-9,9-spirobifluorene, N,N'-bis(phenanthren-9-yl)-N,N'-bis(phenyl)benzidine, 2,2'-bis[N,N-bis(biphenyl-4-yl)amino]-9,9-spirobifluorene, 2,2'-bis(N,N-diphenylamino)-9,9-spirobifluorene, di[4-(N,N-di(p-tolyl)amino)phenyl]cyclohexane, 2,2',7,7'-tetra(N,N-di(p-tolyl))amino-9,9-spirobifluorene, N,N,N',N'-tetranaphthalen-2-ylbenzidine, N,N,N',N'-tetra(3-methylphenyl)-3,3'-dimethylbenzidine, N,N'-di(naphthalenyl)-N,N'-di(naphthalen-2-yl)benzidine, N,N,N',N'-tetra(naphthalenyl)benzidine, N,N'-di(naphthalen-2-yl)-N,N'-diphenylbenzidine-1-4-diamine, N¹,N⁴-diphenyl-N¹,N⁴-di(m-tolyl)benzene-1,4-diamine, N²,N²,N⁶,N⁶-tetraphenylnaphthalene-2,6-diamine, tris(4-(quinolin-8-yl)phenyl)amine, 2,2'-bis(3-(N,N-di(p-tolyl)amino)phenyl)biphenyl, 4,4',4"-tris[3-methylphenyl(phenyl)amino]triphenylamine (m-MTDATA) and 4,4',4"-tris[1-naphthyl(phenyl)amino]triphenylamine (1-TNATA); and
oligothiophenes such as 5,5"-bis-{4-[bis(4-methylphenyl)amino]phenyl}-2,2':5',2"-terthiophene (BMA-3T).

Specific examples of emissive layer-forming materials include tris(8-quinolinolate) aluminum(III) (Alq₃), bis(8-quinolinolate) zinc(II) (Znq₂), bis(2-methyl-8-quinolinolate)-4-(p-phenylphenolate) aluminum(III) (BAlq), 4,4'-bis(2,2-diphenylvinyl)biphenyl, 9,10-di(naphthalen-2-yl)anthracene, 2-t-butyl-9,10-di(naphthalen-2-yl)anthracene, 2,7-bis[9,9-di(4-methylphenyl)fluoren-2-yl]-9,9-di(4-methylphenyl)fluorene, 2-methyl-9,10-bis(naphthalen-2-yl)anthracene, 2-(9,9-spirobifluoren-2-yl)-9,9-spirobifluorene, 2,7-bis(9,9-spirobifluoren-2-yl)-9,9-spirobifluorene, 2-[9,9-di(4-methylphenyl)fluoren-2-yl]-9,9-di(4-methylphenyl)fluorene, 2,2'-dipyrenyl-9,9-spirobifluorene, 1,3,5-tris(pyren-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)phenyl]amine, 10,10'-di(biphenyl-4-yl)-9,9'-bianthracene, N,N'-di(naphthalen-1-yl)-N,N'-diphenyl[1,1':4',1":4",1"'-quaterphenyl]-4,4"'-diamine, 4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluoren-2-yl)pyrene, 1-(7-(9,9'-bianthracen-10-yl)-9,9-dihexyl-9H-fluoren-2-yl)pyrene, 1,3-bis(carbazol-9-yl)benzene, 1,3,5-tris(carbazol-9-yl)benzene, 4,4',4"-tris(carbazol-9-yl)triphenylamine, 4,4'-bis(carbazol-9-yl)biphenyl (CBP), 4,4'-bis(carbazol-9-yl)-2,2'-dimethylbiphenyl, 2,7-bis(carbazol-9-yl)-9,9-dimethylfluorene, 2,2',7,7'-tetrakis(carbazol-9-yl)-9,9-spirobifluorene, 2,7-bis(carbazol-9-yl)-9,9-di(p-tolyl)fluorene, 9,9-bis[4-(carbazol-9-yl)phenyl]fluorene, 2,7-bis(carbazol-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazol-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazole, 9-(4-t-butylphenyl)-3,6-ditrityl-9H-carbazole, 9-(4-t-butylphenyl)-3,6-bis(9-(4-methoxyphenyl)-9H-fluoren-9-yl)-9H-carbazole, 2,6-bis(3-(9H-carbazol-9-yl)phenyl)pyridine, triphenyl(4-(9-phenyl-9H-fluoren-9-yl)phenyl)silane, 9,9-dimethyl-N,N-diphenyl-7-(4-(1-phenyl-1H-benzo[d]imidazol-2-yl)phenyl-9H-fluoren-2-amine, 3,5-bis(3-(9H-carbazol-9-yl)phenyl)pyridine, 9,9-spirobifluoren-2-yldiphenylphosphine oxide, 9,9'-(5-triphenylsilyl)-1,3-phenylene)bis(9H-carbazole), 3-(2,7-bis(diphenylphosphoryl)-9-phenyl-9H-fluoren-9-yl)-9-phenyl-9H-carbazole, 4,4,8,8,12,12-hexa(p-tolyl)-4H-8H-12H-12C-azadibenzo-[cd,mn]pyrene, 4,7-di(9H-carbazol-9-yl)-1,10-phenanthroline, 2,2'-bis(4-(carbazol-9-yl)phenyl)biphenyl, 2,8-bis(diphenylphosphoryl)dibenzo[b,d]thiophene, bis(2-methylphenyl)diphenylsilane, bis[3,5-di(9H-carbazol-9-yl)phenyl]diphenylsilane, 3,6-bis(carbazol-9-yl)-9-(2-ethylhexyl)-9H-carbazole, 3-(diphenylphosphoryl)-9-(4-(diphenylphosphoryl)phenyl)-9H-carbazole and 3,6-bis[(3,5-diphenyl)phenyl]-9-phenylcarbazole. An emissive layer may be formed by co-vapor deposition of any of these materials with a light-emitting dopant.

Specific examples of light-emitting dopants include 3-(2-benzothiazolyl)-7-(diethylamino)coumarin, 2,3,6,7-tetrahydro-1,1,7,7-tetramethyl-1H,5H,11H-10-(2-benzothiazolyl)quinolidino[9,9a,1gh] coumarin, quinacridone, N,N'-dimethylquinacridone, tris(2-phenylpyridine)iridium(III)(Ir(ppy)₃), bis(2-phenylpyridine)(acetylacetonate) iridium(III) (Ir(ppy)₂(acac)), tris[2-(p-tolyl]pyridine)iridium(III)(Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolate] zinc(II), N¹⁰,N¹⁰,N¹⁰,N¹⁰-tetra(p-tolyl)-9,9'-bianthracene-10,10'-diamine, N¹⁰,N¹⁰,N¹⁰,N¹⁰-tetraphenyl-9,9'-bianthracene-10,10'-diamine, N¹⁰,N¹⁰-diphenyl-N¹⁰,N¹⁰-dinaphthalenyl-9,9'-bianthracene-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene. bis[3,5-difluoro-2-(2-pyridyl)phenyl-(2-carboxypyridyl)] iridium(III), 4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)tris(9,9-dimethyl-fluorenylene), 2,7-bis{2-[phenyl(m-tolyl)amino]-9,9-dimethylfluoren-7-yl}-9,9-dimethylfluorene, N-(4-((E)-2-(6((E)-4-(diphenylamino)styryl)naphthalen-2-yl)-vinyl)phenyl)-N-phenylbenzenamine, fac-iridium(III) tris(1-phenyl-3-methylbenzimidazolin-2-ylidene-C,C²), mer-iridium(III) tris(1-phenyl-3-methylbenzimidazolin-2-ylidene-C,C²), 2,7-bis[4-(diphenylamino)styryl]-9,9-spirobifluorene, 6-methyl-2-(4-(9-(4-(6-methylbenzo[d]thiazol-2-yl)phenyl)-anthracen-10-yl)phenyl)benzo[d]thiazole, 1,4-di[4-(N,N-diphenyl)amino]styrylbenzene, 1,4-bis(4-(9H-carbazol-9-yl)styryl)benzene, (E)-6-(4-(diphenylamino)styryl)-N,N-diphenylnaphthalen-2-amine, bis(2,4-difluorophenylpyridinato)(5-(pyridin-2-yl)-1H-tetrazolate) iridium(III), bis(3-trifluoromethyl-5-(2-pyridyl)pyrazole) ((2,4-difluorobenzyl)diphenylphosphinate) iridium(III), bis(3-trifluoromethyl-5-(2-pyridyl)pyrazolate)(benzyl-diphenylphosphinate) iridium(III), bis(1-(2,4-difluorobenzyl)-3-methylbenzimidazolium)(3-(trifluoromethyl)-5-(2-pyridyl)-1,2,4-triazolate) iridium(III), bis(3-trifluoromethyl-5-(2-pyridyl)pyrazolate)(4',6'-difluorophenylpyridinate) iridium(III), bis(4',6'-difluorophenylpyridinato)(3,5-bis(trifluoromethyl)-2-(2'-pyridyl)pyrrolate) iridium(III), bis(4',6'-difluorophenylpyridinato)(3-(trifluoromethyl)-5-(2-pyridyl)-1,2,4-triazolate) iridium (III), (Z)-6-mesityl-N-(6-mesitylquinolin-2(1H)-ylidene)quinoline-2-amine-BF₂, (E)-2-(2-(4-(dimethylamino)styryl)-6-methyl-4H-pyran-4-ylidene)malononitrile, 4-(dicyanomethylene)-2-methyl-6-julolidyl-9-enyl-4-H-pyran, 4-(dicyanomethylene)-2-methyl-6-(1,1,7,7-tetramethyl-julolidyl-9-enyl)-4H-pyran, 4-(dicyanomethylene)-2-t-butyl-6-(1,1,7,7-tetramethyl-julolidin-4-ylvinyl)-4H-pyran, tris(dibenzoylmethane)phenanthroline europium(III), 5,6,11,12-tetraphenylnaphthacene, bis(2-benzo[b]thiophen-2-yl-pyridine) (acetylacetonate) iridium(III), tris(1-phenylisoquinoline) iridium(III), bis(1-phenylisoquinoline)(acetylacetonate) iridium(III), bis[1-(9,9-dimethyl-9H-fluoren-2-yl)isoquinoline]-(acetylacetonate) iridium(III), bis[2-(9,9-dimethyl-9H-fluoren-2-yl)quinoline]-(acetylacetonate) iridium(III), tris[4,4'-di-t-butyl-(2,2')-bipyridine] ruthenium(III)·bis(hexafluorophosphate), tris(2-phenylquinoline) iridium(III), bis(2-phenylquinoline)(acetylacetonate) iridium(III), 2,8-di-t-butyl-5,11-bis(4-t-butylphenyl)-6,12-diphenyltetracene, bis(2-phenylbenzothiazolate)(acetylacetonate) iridium(III), platinum 5,10,15,20-tetraphenyltetrabenzoporphyrin, osmium(II) bis(3-trifluoromethyl-5-(2-pyridine)pyrazolate)-dimethylphenylphosphine, osmium(II) bis(3-trifluoromethyl)-5-(4-t-butylpyridyl)-1,2,4-triazolate)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-t-butylpyridyl)-1,2,4-triazolate)dimethylphenylphosphine, bis[2-(4-n-hexylphenyl)quinoline] (acetylacetonate) iridium(III), tris[2-(4-n-hexylphenyl)quinoline] iridium(III), tris[2-phenyl-4-methylquinoline] iridium(III), bis(2-phenylquinoline) (2-(3-methylphenyl)pyridinate) iridium(III), bis(2-(9,9-diethylfluoren-2-yl)-1-phenyl-1H-benzo[d]-imidazolato)(acetylacetonate) iridium(III), bis(2-phenylpyridine)(3-(pyridin-2-yl)-2H-chromen-9-onate) iridium(III), bis(2-phenylquinoline) (2,2,6,6-tetramethylheptane-3,5-dionate) iridium(III), bis(phenylisoquinoline) (2,2,6,6-tetramethylheptane-3,5-dionate) iridium(III), iridium(III) bis(4-phenylthieno[3,2-c]pyridinato-N,C²)-acetylacetonate, (E)-2-(2-t-butyl-6-(2-(2,6,6-trimethyl-2,4,5,6-tetrahydro-1H-pyrrolo[3,2,1-ij]quinolin-8-yl)vinyl)-4H-pyran-4-ylidene)-malononitrile, bis(3-trifluoromethyl-5-(1-isoquinolyl)pyrazolate)(methyldiphenylphosphine) ruthenium, bis[(4-n-hexylphenyl)isoquinoline] (acetylacetonate) iridium(III), platinum(II) octaethylporphin, bis(2-methyldibenzo[f,h]quinoxaline) (acetylacetonate) iridium(III) and tris[(4-n-hexylphenyl)isoquinoline] iridium(III).

Specific examples of electron transport layer-forming materials include lithium 8-hydroxyquinolinate, 2,2',2"-(1,3,5-benzinetriyl)-tris(1-phenyl-1-H-benzimidazole), 2-(4-biphenyl)-5-(4-t-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine, 3-(4-biphenyl)-4-phenyl-5-t-butylphenyl-1,2,4-triazole, 4-(naphthalen-1-yl)-3,5-diphenyl-4H-1,2,4-triazole, 2,9-bis(naphthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene, 1,3-bis[2-(4-t-butylphenyl)-1,3,4-oxadiazo-5-yl]benzene, tris(2,4,6-trimethyl-3-(pyridin-3-yl)phenyl)borane, 1-methyl-2-(4-(naphthalen-2-yl)phenyl)-1H-imidazo-[4,5f] [1,10]phenanthroline, 2-(naphthalen-2-yl)-4,7-diphenyl-1,10-phenanthroline, phenyldipyrenylphosphine oxide, 3,3',5,5'-tetra[(m-pyridyl)phen-3-yl]biphenyl, 1,3,5-tris[(3-pyridyl)phen-3-yl]benzene, 4,4'-bis(4,6-diphenyl-1,3,5-triazin-2-yl)biphenyl, 1,3-bis[3,5-di(pyridin-3-yl)phenyl]benzene, bis(10-hydroxybenzo[h]quinolinato)beryllium, diphenylbis(4-(pyridin-3-yl)phenyl)silane and 3,5-di(pyren-1-yl)pyridine.

Examples of electron injection layer-forming materials include lithium oxide (Li₂O), magnesium oxide (MgO), alumina (Al₂O₃), lithium fluoride (LiF), sodium fluoride (NaF), magnesium fluoride (MgF₂), cesium fluoride (CsF), strontium fluoride (SrF₂), molybdenum trioxide (MoO₃), aluminum, lithium acetylacetonate Li(acac), lithium acetate and lithium benzoate.

Examples of cathode materials include aluminum, magnesium-silver alloys, aluminum-lithium alloys, lithium, sodium, potassium and cesium.

In cases where the thin-film obtained from the charge-transporting varnish of the invention is a hole injection layer, another example of a method of fabricating the organic EL device of the invention is as follows.

In the EL device fabrication process described above, an organic EL device having a charge-transporting thin-film formed using the charge-transporting varnish of the invention can be fabricated by successively forming a hole-transporting layer and an emissive layer instead of carrying out vacuum evaporation operations for a hole-transporting layer, an emissive layer, an electron-transporting layer and an electron injection layer. Specifically, the charge-transporting varnish of the invention is applied onto an anode substrate, and a hole injection layer is formed by the above-described method. A hole-transporting layer and an emissive layer are then successively formed thereon, following which a cathode material is vapor-deposited on top, thereby giving an organic EL device.

The cathode and anode materials used here may be similar to those described above, and similar cleaning treatment and surface treatment may be carried out.

The method of forming the hole-transporting layer and the emissive layer is exemplified by a film-forming method that entails adding a solvent to a hole-transporting polymer material or a light-emitting polymer material, or to a material obtained by adding a dopant to these, thereby dissolving or uniformly dispersing the material, and then coating the resulting solution or dispersion onto, respectively, the hole injection layer or the hole-transporting layer and subsequently baking.

Examples of hole-transporting polymer materials include poly[(9,9-dihexylfluorenyl-2,7-diyl)-co-(N,N'-bis{p-butylphenyl}-1,4-diaminophenylene)], poly[(9,9-dioctylfluorenyl-2,7-diyl)-co-(N,N'-bis{p-butylphenyl}-1,1'-biphenylene-4,4-diamine)], poly[(9,9-bis{1'-penten-5'-yl}fluorenyl-2,7-diyl)-co-(N,N'-bis{p-butylphenyl}-1,4-diaminophenylene)], poly[N,N'-bis(4-butylphenyl)-N,N'-bis(phenyl)benzidine] end-capped with polysilsesquioxane and poly[(9,9-dioctylfluorenyl-2,7-diyl)-co-(4,4'-(N-(p-butylphenyl))diphenylamine)].

Examples of light-emitting polymer materials include polyfluorene derivatives such as poly(9,9-dialkylfluorene) (PDAF), poly(phenylene vinylene) derivatives such as poly(2-methoxy-5-(2'-ethylhexoxy)-1,4-phenylene vinylene) (MEH-PPV), polythiophene derivatives such as poly(3-alkylthiophene) (PAT), and polyvinylcarbazole (PVCz).

Examples of the solvent include toluene, xylene and chloroform. Examples of the method of dissolution or uniform dispersion include stirring, stirring under applied heat, and ultrasonic dispersion.

Examples of the coating method include, but are not particularly limited to, inkjet printing, spraying, dipping, spin coating, transfer printing, roll coating and brush coating. Coating is preferably carried out in an inert gas atmosphere such as nitrogen or argon.

Examples of the baking method include methods that involve heating in an oven or on a hot plate, either within an inert gas atmosphere or in a vacuum.

An example is described below of a method of fabricating the organic EL device of the invention in a case where the thin-film obtained from the charge-transporting varnish of the invention is a hole-transporting layer.

A hole injection layer is formed on an anode substrate. The charge-transporting varnish of the invention is coated onto this layer and baked by the above-described method, thereby producing a hole-transporting layer. An emissive layer, an electron-transporting layer, an electron injection layer and a cathode are provided in this order on the hole-transporting layer. Methods of forming the emissive layer, electron-transporting layer and electron injection layer, and specific examples of each, are exemplified in the same way as above. The hole injection layer may be formed by any vapor deposition process or coating process (wet process), according to the properties, etc. of the material used.

Illustrative examples of the material that forms the hole injection layer include copper phthalocyanine, titanium oxide phthalocyanine, platinum phthalocyanine, pyrazino[2,3-f][1,10]phenanthroline-2,3-dicarbonitrile, N,N,N',N'-tetrakis(4-methoxyphenyl)benzidine, 2,7-bis[N,N-bis(4-methoxyphenyl)amino]-9,9-spirobifluorene, 2,2'-bis[N,N-bis(4-methoxyphenyl)amino]-9,9-spirobifluorene, N,N'-diphenyl-N,N'-di[4-(N,N-ditolylamino)phenyl]benzidine, N,N'-diphenyl-N,N'-di[4-(N,N-diphenylamino)phenyl]benzidine, N⁴,N^{4'}-(biphenyl-4,4'-diyl)bis(N⁴,N^{4'},N^{4'}-triphenylbiphenyl-4,4'-diamine), N¹,N^{1'}-(biphenyl-4,4'-diyl)bis(N¹-phenyl-N⁴,N^{4'}-di-m-tolylbenzene-1,4-diamine), and the charge-transporting materials mentioned in WO 2004/043117, WO 2004/105446, WO 2005/000832, WO 2005/043962, WO 2005/042621, WO 2005/107335, WO 2006/006459, WO 2006/025342, WO 2006/137473, WO 2007/049631, WO 2007/099808, WO 2008/010474, WO 2008/032617, WO 2008/032616, WO 2008/129947, WO 2009/096352, WO 2010/041701, WO 2010/058777, WO 2010/058776, WO 2013/042623, WO 2013/129249, WO 2014/115865, WO 2014/132917, WO 2014/141998, and WO 2014/132834.

The anode material, the materials which form the emissive layer, the light-emitting dopant, the electron-transporting layer and the electron-blocking layer, and the cathode material are exemplified in the same way as above.

An example is described below of a method of fabricating the organic EL device of the invention in a case where the thin-film obtained from the charge-transporting varnish of the invention is a hole injecting and transporting layer.

A hole injecting and transporting layer is formed on an anode substrate, and an emissive layer, an electron-transporting layer, an electron injection layer and a cathode are provided in this order on the hole injecting and transporting layer. Methods of forming the emissive layer, electron-transporting layer and electron injection layer, and specific examples of each, are exemplified in the same way as above.

The anode material, the materials which form the emissive layer, the light-emitting dopant, the electron-transporting layer and the electron-blocking layer, and the cathode material are exemplified in the same way as above.

A hole-blocking layer, an electron-blocking layer or the like may be optionally provided between the electrodes and any of the above layers. By way of illustration, an example of a material that forms an electron-blocking layer is tris(phenylpyrazole)iridium.

The materials which make up the anode, the cathode and the layers formed therebetween differ according to whether a device provided with a bottom emission structure or a top emission structure is to be fabricated, and so are suitably selected while taking this into account.

Generally, in an element having a bottom emission structure, a transparent anode is used on the substrate side and light is extracted from the substrate side, whereas in an element having a top emission structure, a reflective anode made of metal is used and light is extracted from a transparent electrode (cathode) side in the opposite direction from the substrate. Hence, with regard to the anode material, for example, when fabricating a device having a bottom emission structure, a transparent anode of ITO or the like is used, and when manufacturing a device having a top emission structure, a reflective anode of Al/Nd or the like is used.

The organic EL device of the invention, in order to prevent a deterioration in characteristics, may be sealed in the usual manner with, if necessary, a desiccant or the like.

### EXAMPLES

Working Examples are given below to more concretely illustrate the invention, although the invention is not limited by these Examples. The equipment used was as follows.
(1) ¹H-NMR Measurement:
   JNM-ECP300 FT NMR System, from JEOL Ltd.
(2) LC/MS:
   ZQ 2000 mass spectrometer, from Waters Corporation
(3) Substrate Cleaning:
   Substrate cleaning machine (reduced-pressure plasma system), from Choshu Industry Co., Ltd.
(4) Varnish Coating:
   MS-A100 Spin Coater, from Mikasa Co., Ltd.
(5) Film Thickness Measurement:
   Surfcorder ET-4000 microfigure measuring instrument, from Kosaka Laboratory, Ltd.
(6) EL Device Fabrication:
   C-E2L1G1-N Multifunction Vapor Deposition System, from Choshu Industry Co., Ltd.
(7) Measurement of EL Device Brightness:
   I-V-L Measurement System from Tech World, Inc.
(8) Measurement of Transmittance:
   UV-3100PC visible-UV spectrophotometer,
   from Shimadzu Corporation
(9) Measurement of Ionization Potential:
   AC-3, from Riken Keiki Co., Ltd.

### [1] Synthesis of Compounds

### [Synthesis Example 1] Synthesis of Compound 1

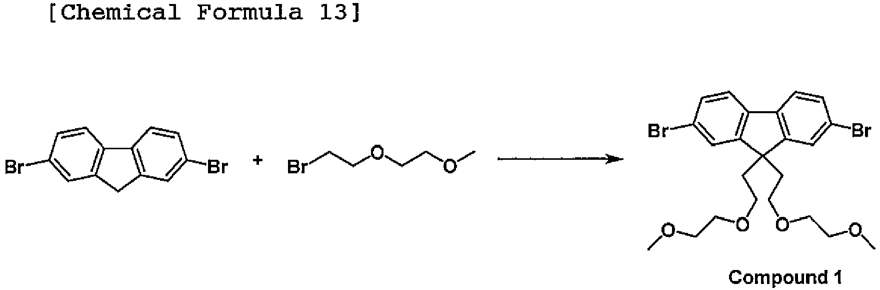

Potassium hydroxide (5.61 g, 100 mmol), potassium iodide (0.33 g, 2 mmol) and 1-bromo-2-(2-methoxyethoxy)ethane (8.05 g, 44 mmol) were added to a dimethylsulfoxide suspension (130 mL) of 2,7-dibromofluorene (6.48 g, 20 mmol; available from Tokyo Chemical Industry Co., Ltd.), and the system was stirred at room temperature for 24 hours. Following reaction completion, the system was cooled to 0°C, water (120 mL) was added, and neutralization was carried out with hydrochloric acid. The crude product obtained by extracting the organic layer with ethyl acetate, drying over magnesium sulfate and concentration was purified by silica gel column chromatography (eluate: hexane/ethyl acetate (4/1 → 3/1)), giving 8.47 g (80% yield) of Compound 1 as a yellow liquid. The ¹H-NMR and LC/MS results were as follows.
¹H-NMR (300 MHz, CDCl₃):
δ 2.36 (app t; J=7.2 Hz, 4H), 2.78 (app t, J=7.2 Hz, 4H), 3.17-3.20 (m, 4H), 3.28-3.31 (m, 10H), 7.43-7.53 (m, 4H), 7.57 (dd, J=1.8, 14.7 Hz, 2H)
LC/MS (ESI⁺) m/z; 529 [M+1]⁺

### [Synthesis Example 2] Synthesis of Compound 2

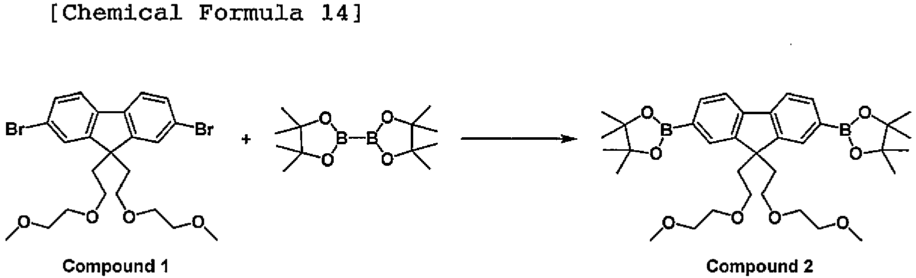

Potassium acetate (3.32 g, 33.8 mmol), the dichloromethane adduct of PdCl₂(dppf) (0.35 g, 0.42 mmol) and 1,4-dioxane (15 mL) were added to a 1,4-dioxane solution (30 mL) of Compound 1 (4.47 g, 8.5 mmol) prepared in Synthesis Example 1 and bis(pinacolato)diborane (4.73 g, 18.6 mmol), and the system was flushed with nitrogen, then heated at 100°C for 5 hours.

Following reaction completion, the crude product obtained by Celite® filtration and concentration of the filtrate was purified by silica gel column chromatography (eluate: hexane/ethyl acetate (4/1 → 2/1)), giving 1.97 g (37% yield) of Compound 2 as a colorless solid. The ¹H-NMR and LC/MS results were as follows.
¹H-NMR (300 MHz, CDCl₃):
δ 1.39 (s, 24H), 2.47 (app t, J=7.2 Hz, 4H), 2.68 (app t, J=7.2 Hz, 4H), 3.14-3.18 (m, 4H), 3.27-3.30 (m, 10H), 7.70 (d, J=7.5 Hz, 2H), 7.80 (d, J=7.8 Hz, 2H), 7.86 (s, 2H).
LC/MS (ESI⁺) m/z; 529 [M+1]⁺

### [Synthesis Example 3] Synthesis of Compound 3

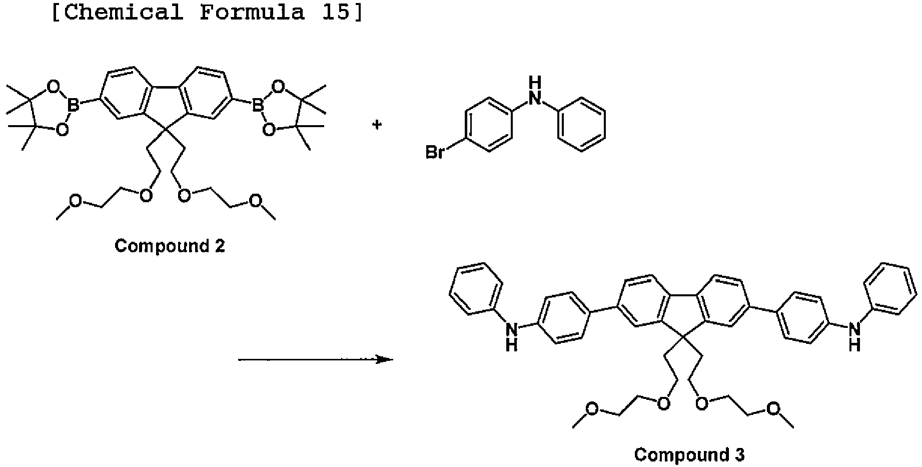

1,4-Dioxane (25 mL) and water (6 mL) were added to Compound 2 (1.24 g, 2 mmol) prepared in Synthesis Example 2, 4-bromodiphenylamine (1.09 g, 4.4 mmol), potassium carbonate (1.11 g, 8 mmol) and Pd(PPh₃)₄ (46.2 mg, 0.04 mmol), and the system was flushed with nitrogen, then heated at 90°C for 6 hours.

Following reaction completion, Celite filtration was carried out, and the organic layer of the filtrate was extracted with ethyl acetate. The crude product obtained by drying the extract over sodium sulfate and concentration was purified by silica gel column chromatography (eluate: hexane/ethyl acetate (9/1 → 4/1 → 3/1 → 2/1 → 3/2 → 1/1)), giving 0.52 g (37% yield) of Compound 3 as a colorless solid. The ¹H-NMR and LC/MS results were as follows.
¹H-NMR (300 MHz, CDCl₃):
δ 2.49 (app t, J=7.2 Hz, 4H), 2.86 (app t, J=7.2 Hz, 4H), 3.19-3.31 (m, 14H), 5.83 (brs, 2H), 6.97 (t, J=7.2 Hz, 2H), 7.12-7.19 (m, 8H), 7.28-7.33 (m, 4H), 7.56-7.62 (m, 8H), 7.72 (d, J=7.8 Hz, 2H).
LC/MS (ESI⁺) m/z; 705 [M+1]⁺

### [Synthesis Example 4] Synthesis of Compound 4

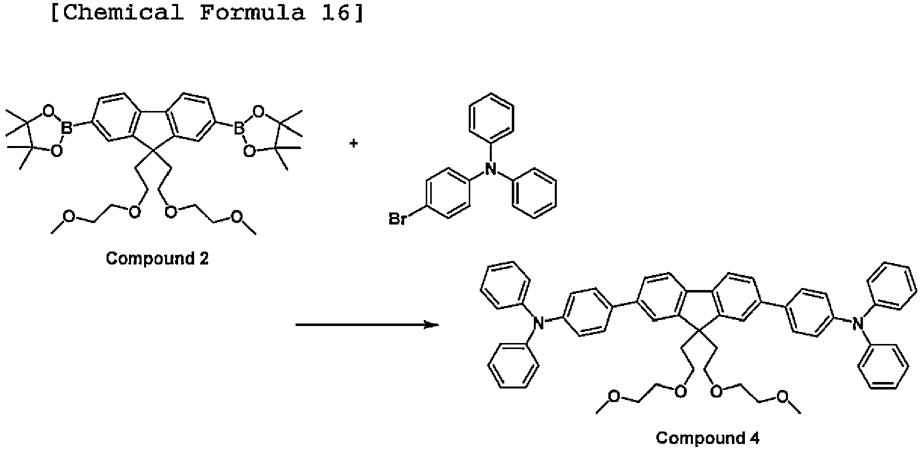

Using 0.66 g (1.1 mmol) of Compound 2 prepared in Synthesis Example 2, 4-bromotriphenylamine (0.76 g, 2.3 mmol), potassium carbonate (0.59 g, 4.2 mmol) and Pd(PPh₃)₄ (61.2 mg, 0.05 mmol), synthesis was carried out in the same way as in Synthesis Example 3, giving 0.43 g (47% yield) of Compound 4 as a light-yellow solid. The ¹H-NMR and LC/MS results were as follows.
¹H-NMR (300 MHz, CDCl₃):
δ 2.48 (app t, J=7.5 Hz, 4H), 2.84 (app t, J=7.5 Hz, 4H), 3.18-3.31 (m, 14H), 7.04 (t, J=7.2 Hz, 4H), 7.14-7.19 (m, 8H), 7.28-7.31 (m, 12H), 7.54-7.62 (m, 8H), 7.72 (d, J=7.8 Hz, 2H).
LC/MS (ESI⁺) m/z; 857 [M+1]⁺

### [Synthesis Example 5] Synthesis of Compound 5

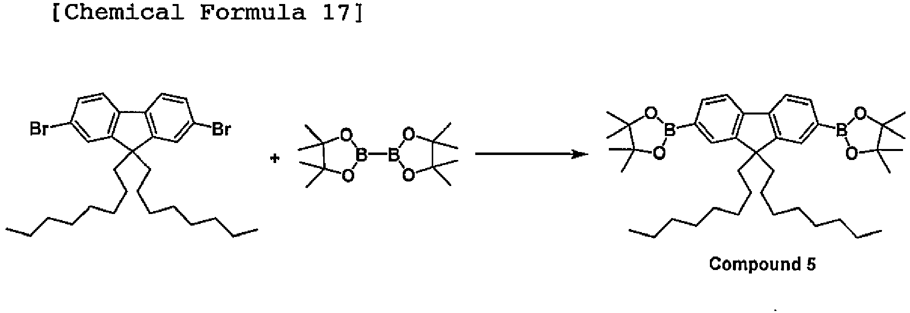

Using 9,9-dioctyl-2,7-dibromofluorene (3.29 g, 6 mmol; from Aldrich Co.), bis(pinacolato)diborane (3.35 g, 13.2 mmol), potassium acetate (2.36 g, 24 mmol) and the dichloromethane adduct of PdCl₂(dppf) (0.20 g, 0.24 mmol), synthesis was carried out in the same way as in Synthesis Example 2, giving 3.29 g (85% yield) of Compound 5 as a colorless solid. The ¹H-NMR results were as follows.
¹H-NMR (300 MHz, CDCl₃).
δ 0.55 (brs, 4H), 0.81 (t, J=7.2 Hz, 6H), 1.01-1.22 (m, 20H), 1.39 (s, 24H), 1.97-2.02 (m, 4H), 7.70-7.74 (m, 4H), 7.80 (d, J=7.2 Hz, 2H).

### [Synthesis Example 6] Synthesis of Compound 6 (Not according to the invention)

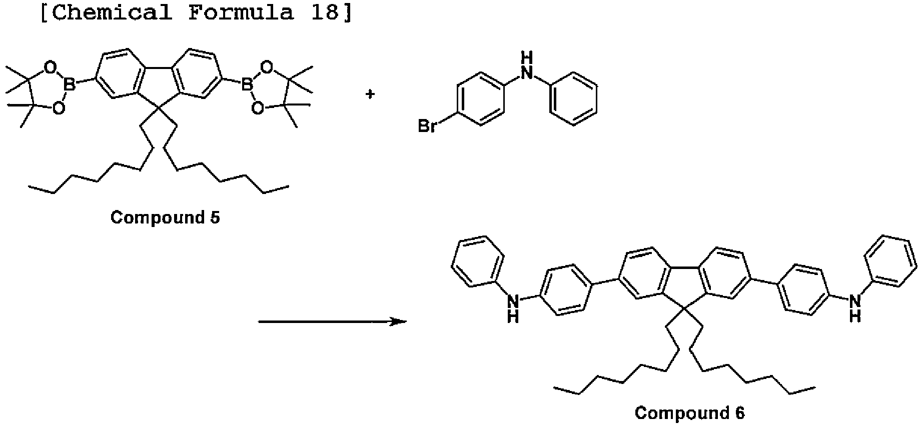

Using 1.29 g (2 mmol) of Compound 5 prepared in Synthesis Example 5, 4-bromodiphenylamine (1.09 g, 4.4 mmol), potassium carbonate (1.11 g, 8 mmol) and Pd(PPh₃)₄ (46.2 mg, 0.04 mmol), synthesis was carried out in the same way as in Synthesis Example 3, giving 0.95 g (66% yield) of Compound 6 as a colorless solid. The ¹H-NMR and LC/MS results were as follows.
¹H-NMR (300 MHz, CDCl₃):
δ 0.78-0.83 (m, 10H), 1.07-1.20 (m, 20H), 2.01-2.03 (m, 4H), 5.80 (brs, 2H), 6.96 (t, J=7.2 Hz, 2H), 7.12-7.20 (m, 8H), 7.28-7.33 (m, 4H), 7.54-7.62 (m, 8H), 7.74 (d, J=8.1 Hz, 2H).
LC/MS (ESI⁺) m/z; 725 [M+1]⁺

### [2] Preparation of Charge-Transporting Varnishes

### [Working Example 1-1]

A charge-transporting varnish was prepared by, under a nitrogen atmosphere: dissolving 0.045 g of Compound 3 in 3.5 g of 1,3-dimethyl-2-imidazolidinone, adding 0.5 g of cyclohexanol and 0.5 g of propylene glycol to the resulting solution, and stirring.

### [Working Example 1-2, Comparative Example 1-1]

Aside from using Compound 4 (Working Example 1-2) or Compound 6 (Comparative Example 1-1) instead of Compound 3, charge-transporting varnishes were prepared in the same way as in Working Example 1-1.

### [Working Example 1-3]

A charge-transporting varnish was prepared by, under a nitrogen atmosphere: dissolving 0.037 g of Compound 3, 0.051 g of the arylsulfonic acid of formula (S1) and 0.022 g of phosphotungstic acid in 4.2 g of 1,3-dimethyl-2-imidazolidinone, then adding 0.6 g of cyclohexanol and 0.6 g of propylene glycol to the resulting solution and stirring.

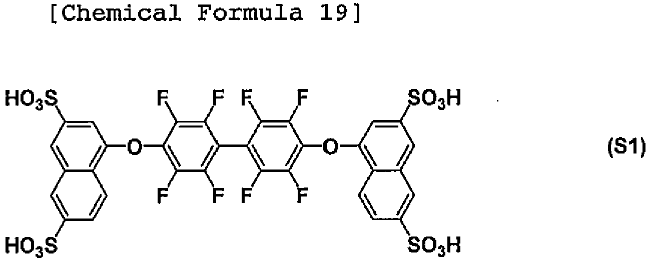

### [Working Example 1-4, Comparative Example 1-2]

Aside from using Compound 4 (Working Example 1-4) or Compound 6 (Comparative Example 1-2) instead of Compound 3, charge-transporting varnishes were prepared in the same way as in Working Example 1-3.

### [3] Fabrication of Organic EL Devices and Evaluation of Device Characteristics

### [Working Example 2-1]

The varnish obtained in Working Example 1-2 was coated onto an ITO substrate using a spin coater, then dried for 5 minutes at 80°C and subsequently baked for 10 minutes at 230°C in an open-air atmosphere, thereby forming a uniform 30 nm thin-film on an ITO substrate. A glass substrate with dimensions of 25 mm × 25 mm × 0.7 mm (t) and having indium-tin oxide (ITO) patterned on the surface to a film thickness of 150 nm was used as the ITO substrate. Prior to use, impurities on the surface were removed with an O₂ plasma cleaning system (150 W, 30 seconds).

Next, using a vapor deposition system (degree of vacuum, 1.0×10⁻⁵ Pa), thin-films of Alq₃, lithium fluoride and aluminum were successively deposited onto the ITO substrate on which a thin-film had been formed, thereby giving an organic EL device. At this time, vapor deposition was carried out at a rate of 0.2 nm/s for Alq₃ and aluminum, and at a rate of 0.02 nm/s for lithium fluoride. The film thicknesses were set to, respectively, 40 nm, 0.5 nm and 120 nm.

To prevent the device characteristics from deteriorating due to the influence of oxygen, moisture and the like in air, the organic EL device was sealed with sealing substrates, after which the device characteristics were evaluated. Sealing was carried out by the following procedure.

In a nitrogen atmosphere having an oxygen concentration of not more than 2 ppm and a dew point of not more than -85°C, the organic EL device was placed between sealing substrates and the sealing substrates were laminated together using an adhesive (MORESCO Moisture Cut WB90US(P), from Moresco Corporation). At this time, a desiccant (HD-071010W-40, from Dynic Corporation) was placed, together with the organic EL device, within the sealing substrates. The laminated sealing substrates were irradiated with UV light (wavelength, 365 nm; dosage, 6,000 mJ/cm²), and then annealed at 80°C for 1 hour to cure the adhesive.

### [Working Example 2-2]

Aside from using the varnish obtained in Working Example 1-3 instead of the varnish obtained in Working Example 1-2 and using a vapor deposition system (degree of vacuum, 1.0×10⁻⁵ Pa) to form a thin-film of α-NPD between the thin-film formed using this varnish and a thin-film of Alq₃, an organic EL device was fabricated in the same way as in Working Example 2-1. The vapor deposition rate for α-NPD was set to 0.02 nm/s and the film thickness was set to 30 nm.

### [Comparative Examples 2-1 and 2-2]

Aside from using the varnish obtained in Comparative Example 1-1 or 1-2 instead of the varnish obtained in Working Example 1-2, organic EL devices were fabricated in the same way as in Working Example 2-1.

### [Comparative Examples 2-3 and 2-4]

Aside from using the varnish obtained in Comparative Example 1-1 or 1-2 instead of the varnish obtained in Working Example 1-3, organic EL devices were fabricated in the same way as in Working Example 2-2.

The current density and brightness at a driving voltage of 6 V were measured for these fabricated devices. The results are shown in Table 1. The size (area) of the light-emitting surface in each device was 2 mm × 2 mm (the same applies below).

**[Table 1]**

| | Current density (mA/cm²) | Brightness (cd/m²) |
|---|---|---|
| Working Example 2-1 | 127 | 2,190 |
| Working Example 2-2 | 29 | 1,150 |
| Comparative Example 2-1 | 45 | 49 |
| Comparative Example 2-2 | 25 | 2.07 |
| Comparative Example 2-3 | 0.02 | 0.58 |
| Comparative Example 2-4 | 6 | 161 |

As shown in Table 1, by using a thin-film obtained from a charge-transporting varnish of the invention as the hole injection layer or hole injecting and transporting layer, organic EL devices having excellent brightness characteristics are obtained.

### [4] Measurement of Thin-Film Transmittance

### [Working Examples 3-1 to 3-4]

The varnishes obtained in Working Examples 1-1 to 1-4 were each coated onto a quartz substrate using a spin coater, after which they were dried for 1 minute at 80°C in an open-air atmosphere and subsequently baked at 230°C for 15 minutes, thereby forming in each case a uniform thin-film having a thickness of 30 nm on the quartz substrate. The transmittance of the thin-film thus formed was then measured. The transmittance was obtained by scanning over the visible range; that is, over wavelengths of 400 to 800 nm. The average transmittance for 400 to 800 nm is shown in Table 2. The quartz substrate was used after removing impurities on the surface with a plasma cleaning system (150 W, 30 seconds).

**[Table 2]**

| | Transmittance (%) |
|---|---|
| Working Example 3-1 | 96 |
| Working Example 3-2 | 96 |
| Working Example 3-3 | 97 |
| Working Example 3-4 | 95 |

As shown in Table 2, thin-films obtained from the charge-transporting varnishes of the invention also had excellent transmittances.

### [5] Measurement of Ionization Potential (Ip)

The ionization potentials of the thin-films produced in Working Examples 3-1 to 3-3 were measured. The results are shown in Table 3.

**[Table 3]**

| | Ip (eV) |
|---|---|
| Working Example 3-1 | 5.34 |
| Working Example 3-2 | 5.54 |
| Working Example 3-3 | 5.69 |

As shown in Table 3, the highest occupied molecular orbital (HOMO) level of the thin-film in Working Example 3-2 in particular is close to the HOMO level (5.5 eV) of a vapor-deposited film of the hole-transporting material α-NPD. Hence, this thin-film is expected to have an excellent hole transporting ability to an emissive layer (e.g., the Alq₃ vapor-deposited film in Working Example 2-1). The HOMO level of the thin-film in Working Example 3-3 is deeper than the HOMO level of the α-NPD vapor-deposited film, and so this thin-film is expected to have an excellent hole injecting ability to the hole-transporting layer.

## Claims

1. A fluorene derivative **characterized by** having formula (1) 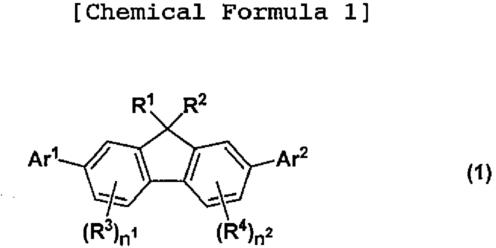
wherein R¹ and R² are each independently a hydrogen atom, an alkyl group of 1 to 20 carbon atoms, an alkenyl group of 2 to 20 carbon atoms, an alkynyl group of 2 to 20 carbon atoms, an aryl group of 6 to 20 carbon atoms, a heteroaryl group of 2 to 20 carbon atoms, an alkoxy group of 1 to 20 carbon atoms, an alkenyloxy group of 2 to 20 carbon atoms, an alkynyloxy group of 2 to 20 carbon atoms, an aryloxy group of 6 to 20 carbon atoms, a heteroaryloxy group of 2 to 20 carbon atoms, or an alkyl group of 2 to 20 carbon atoms having at least one ether structure (with the proviso that at least one of R¹ and R² is said alkoxy group, alkenyloxy group, alkynyloxy group, aryloxy group, heteroaryloxy group, or alkyl group having at least one ether structure);
R³ and R⁴ are each independently a halogen atom, a nitro group, a cyano group, an alkyl group of 1 to 20 carbon atoms which may be substituted with Z¹, an alkenyl group of 2 to 20 carbon atoms which may be substituted with Z¹, an alkynyl group of 2 to 20 carbon atoms which may be substituted with Z¹, an alkoxy group of 1 to 20 carbon atoms which may be substituted with Z¹, an alkenyloxy group of 2 to 20 carbon atoms which may be substituted with Z¹, an alkynyloxy group of 2 to 20 carbon atoms which may be substituted with Z¹, an aryl group of 6 to 20 carbon atoms which may be substituted with Z², a heteroaryl group of 2 to 20 carbon atoms which may be substituted with Z², an aryloxy group of 6 to 20 carbon atoms which may be substituted with Z², or a heteroaryloxy group of 2 to 20 carbon atoms which may be substituted with Z², the respective R³ groups and the respective R⁴ groups being mutually the same or different;
Z¹ is a halogen atom, a nitro group, a cyano group, an aryl group of 6 to 20 carbon atoms which may be substituted with Z³, a heteroaryl group of 2 to 20 carbon atoms which may be substituted with Z³, an alkoxy group of 1 to 20 carbon atoms which may be substituted with Z³, an alkenyloxy group of 2 to 20 carbon atoms which may be substituted with Z³, an alkynyloxy group of 2 to 20 carbon atoms which may be substituted with Z³, an aryl group of 6 to 20 carbon atoms which may be substituted with Z³, or a heteroaryl group of 2 to 20 carbon atoms which may be substituted with Z³;
Z² is a halogen atom, a nitro group, a cyano group, an alkyl group of 1 to 20 carbon atoms which may be substituted with Z³, an alkenyl group of 2 to 20 carbon atoms which may be substituted with Z³, an alkynyl group of 2 to 20 carbon atoms which may be substituted with Z³, an alkoxy group of 1 to 20 carbon atoms which may be substituted with Z³, an alkenyloxy group of 2 to 20 carbon atoms which may be substituted with Z³, an alkynyloxy group of 2 to 20 carbon atoms which may be substituted with Z³, an aryl group of 6 to 20 carbon atoms which may be substituted with Z³, or a heteroaryl group of 2 to 20 carbon atoms which may be substituted with Z³;
Z³ is a halogen atom, a nitro group or a cyano group;
n¹ and n² represent the number of, respectively, R³ substituents and R⁴ substituents, and are each independently an integer from 0 to 3; and
Ar¹ and Ar² are each independently a group having any of formulas (A1) to (A13) 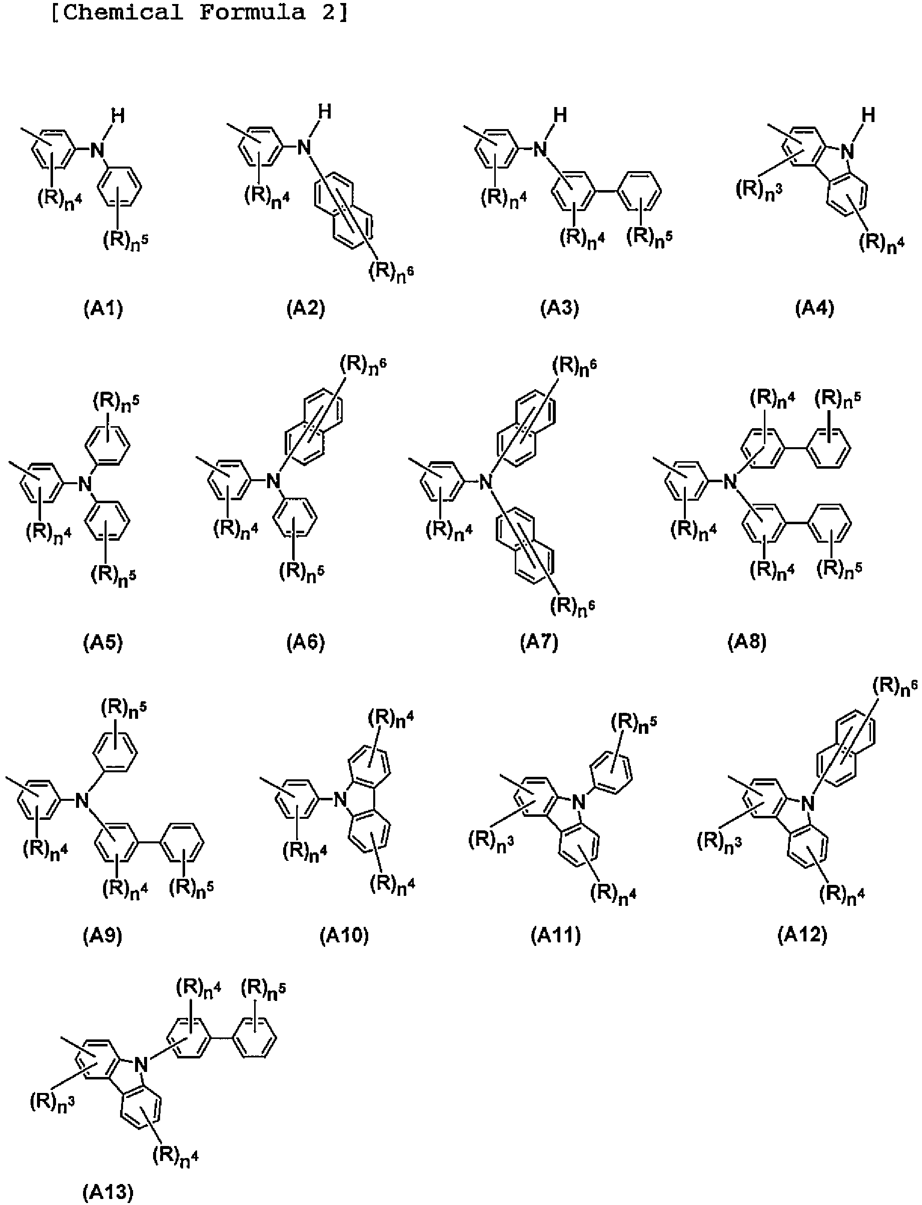 (wherein R is a halogen atom, a nitro group, a cyano group, an alkyl group of 1 to 20 carbon atoms which may be substituted with Z³, an alkenyl group of 2 to 20 carbon atoms which may be substituted with Z³, an alkynyl group of 2 to 20 carbon atoms which may be substituted with Z³, an alkoxy group of 1 to 20 carbon atoms which may be substituted with Z¹, an alkenyloxy group of 2 to 20 carbon atoms which may be substituted with Z³, or an alkynyloxy group of 2 to 20 carbon atoms which may be substituted with Z³, the respective R groups being the same or different; and
n³ to n⁶ represent the number of R substituents, n³ being an integer from 0 to 3, n⁴ being an integer from 0 to 4, n⁵ being an integer from 0 to 5 and n⁶ being an integer from 0 to 7, with each of n³ to n⁶ being the same or different;
wherein alkyl group, alkenyl group, alkynyl group, alkyloxy group, alkenyloxy group and alkynyloxy group may be linear, branched or cyclic.

2. The fluorene derivative of claim 1, wherein R¹ and R² are both an alkyl group of 2 to 20 carbon atoms which includes at least one ether structure.

3. The fluorene derivative of claim 1 or 2, wherein n¹ and n² are both 0.

4. A charge-transporting substance consisting of the fluorene derivative of any one of claims 1 to 3.

5. A charge-transporting varnish comprising the charge-transporting substance of claim 4 and an organic solvent.

6. The charge-transporting varnish of claim 5 which further comprises a dopant substance.

7. A charge-transporting thin-film produced using the charge-transporting varnish of claim 5 or 6.

8. An organic electroluminescent device comprising the charge-transporting thin-film of claim 7.

9. A method of preparing the fluorene derivative of claim 1, which method is **characterized by** comprising the step of carrying out a cross-coupling reaction between a boric acid ester compound of formula (1") or (1"') and compounds of formula (A') and (A") in the presence of a catalyst 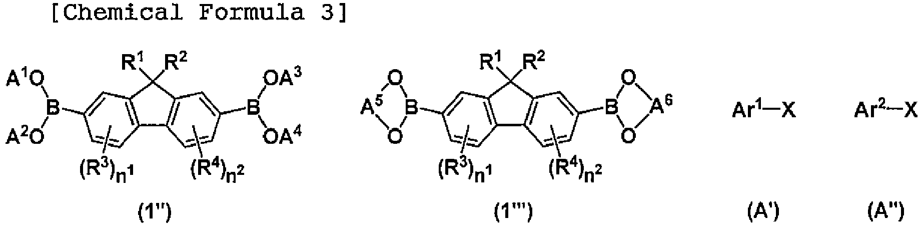 (wherein R¹ to R⁴, Ar¹, Ar², n¹ and n² are as defined in claim 1; each X is independently a halogen atom or a pseudo-halogen group; A¹ to A⁴ are each independently a hydrogen atom, an alkyl group of 1 to 20 carbon atoms or an aryl group of 6 to 20 carbon atoms; and A⁵ and A⁶ are each independently an alkanediyl group of 1 to 20 carbon atoms or an arylene group of 6 to 20 carbon atoms).

## Patentansprüche

1. Fluorenderivat, **dadurch gekennzeichnet, dass** es die Formel (1) aufweist: 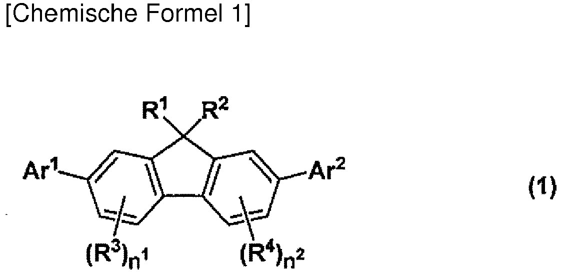
worin: R¹ und R² jeweils unabhängig voneinander ein Wasserstoffatom, eine Alkylgruppe mit 1 bis 20 Kohlenstoffatomen, eine Alkenylgruppe mit 2 bis 20 Kohlenstoffatomen, eine Alkinylgruppe mit 2 bis 20 Kohlenstoffatomen, eine Arylgruppe mit 6 bis 20 Kohlenstoffatomen, eine Heteroarylgruppe mit 2 bis 20 Kohlenstoffatomen, eine Alkoxygruppe mit 1 bis 20 Kohlenstoffatomen, eine Alkenyloxygruppe mit 2 bis 20 Kohlenstoffatomen, eine Alkinyloxygruppe mit 2 bis 20 Kohlenstoffatomen, eine Aryloxygruppe mit 6 bis 20 Kohlenstoffatomen, eine Heteroaryloxygruppe mit 2 bis 20 Kohlenstoffatomen oder eine Alkylgruppe mit 2 bis 20 Kohlenstoffatomen sind, die zumindest eine Etherstruktur aufweist (mit der Maßgabe, dass zumindest einer von R¹ und R² die Alkoxygruppe, Alkenyloxygruppe, Alkinyloxygruppe, Aryloxygruppe, Heteroaryloxygruppe oder Alkylgruppe ist, die zumindest eine Etherstruktur aufweist);
R³ und R⁴ jeweils unabhängig voneinander ein Halogenatom, eine Nitrogruppe, eine Cyanogruppe, eine gegebenenfalls mit Z¹ substituierte Alkylgruppe mit 1 bis 20 Kohlenstoffatomen, eine gegebenenfalls mit Z¹ substituierte Alkenylgruppe mit 2 bis 20 Kohlenstoffatomen, eine gegebenenfalls mit Z¹ substituierte Alkinylgruppe mit 2 bis 20 Kohlenstoffatomen, eine gegebenenfalls mit Z¹ substituierte Alkoxygruppe mit 1 bis 20 Kohlenstoffatomen, eine gegebenenfalls mit Z¹ substituierte Alkenyloxygruppe mit 2 bis 20 Kohlenstoffatomen, eine gegebenenfalls mit Z¹ substituierte Alkinyloxygruppe mit 2 bis 20 Kohlenstoffatomen, eine gegebenenfalls mit Z² substituierte Arylgruppe mit 6 bis 20 Kohlenstoffatomen, eine gegebenenfalls mit Z² substituierte Heteroarylgruppe mit 2 bis 20 Kohlenstoffatomen, eine gegebenenfalls mit Z² substituierte Aryloxygruppe mit 6 bis 20 Kohlenstoffatomen oder eine gegebenenfalls mit Z² substituierte Heteroaryloxygruppe mit 2 bis 20 Kohlenstoffatomen sind, wobei die jeweiligen Gruppen R³ und die jeweiligen Gruppen R⁴ jeweils gleich oder unterschiedlich sein können;
Z¹ ein Halogenatom, eine Nitrogruppe, eine Cyanogruppe, eine gegebenenfalls mit Z³ substituierte Arylgruppe mit 6 bis 20 Kohlenstoffatomen, eine gegebenenfalls mit Z³ substituierte Heteroarylgruppe mit 2 bis 20 Kohlenstoffatomen, eine gegebenenfalls mit Z³ substituierte Alkoxygruppe mit 1 bis 20 Kohlenstoffatomen, eine gegebenenfalls mit Z³ substituierte Alkenyloxygruppe mit 2 bis 20 Kohlenstoffatomen, eine gegebenenfalls mit Z³ substituierte Alkinyloxygruppe mit 2 bis 20 Kohlenstoffatomen, eine gegebenenfalls mit Z³ substituierte Arylgruppe mit 6 bis 20 Kohlenstoffatomen oder eine gegebenenfalls mit Z³ substituierte Heteroarylgruppe mit 2 bis 20 Kohlenstoffatomen;
Z² ein Halogenatom, eine Nitrogruppe, eine Cyanogruppe, eine gegebenenfalls mit Z³ substituierte Alkylgruppe mit 1 bis 20 Kohlenstoffatomen, eine gegebenenfalls mit Z³ substituierte Alkenylgruppe mit 2 bis 20 Kohlenstoffatomen, eine gegebenenfalls mit Z³ substituierte Alkinylgruppe mit 2 bis 20 Kohlenstoffatomen, eine gegebenenfalls mit Z³ substituierte Alkoxygruppe mit 1 bis 20 Kohlenstoffatomen, eine gegebenenfalls mit Z³ substituierte Alkenyloxygruppe mit 2 bis 20 Kohlenstoffatomen, eine gegebenenfalls mit Z³ substituierte Alkinyloxygruppe mit 2 bis 20 Kohlenstoffatomen, eine gegebenenfalls mit Z³ substituierte Arylgruppe mit 6 bis 20 Kohlenstoffatomen oder eine gegebenenfalls mit Z³ substituierte Heteroarylgruppe mit 2 bis 20 Kohlenstoffatomen;
Z³ ein Halogenatom, eine Nitrogruppe oder eine Cyanogruppe ist;
n¹ und n² für die Anzahl an Substituenten R³ bzw. Substituenten R⁴ stehen und jeweils unabhängig voneinander eine ganze Zahl von 0 bis 3 sind; und
Ar¹ und Ar² jeweils unabhängig voneinander eine Gruppe sind, die eine der Formeln (A1) bis (A13) aufweist: 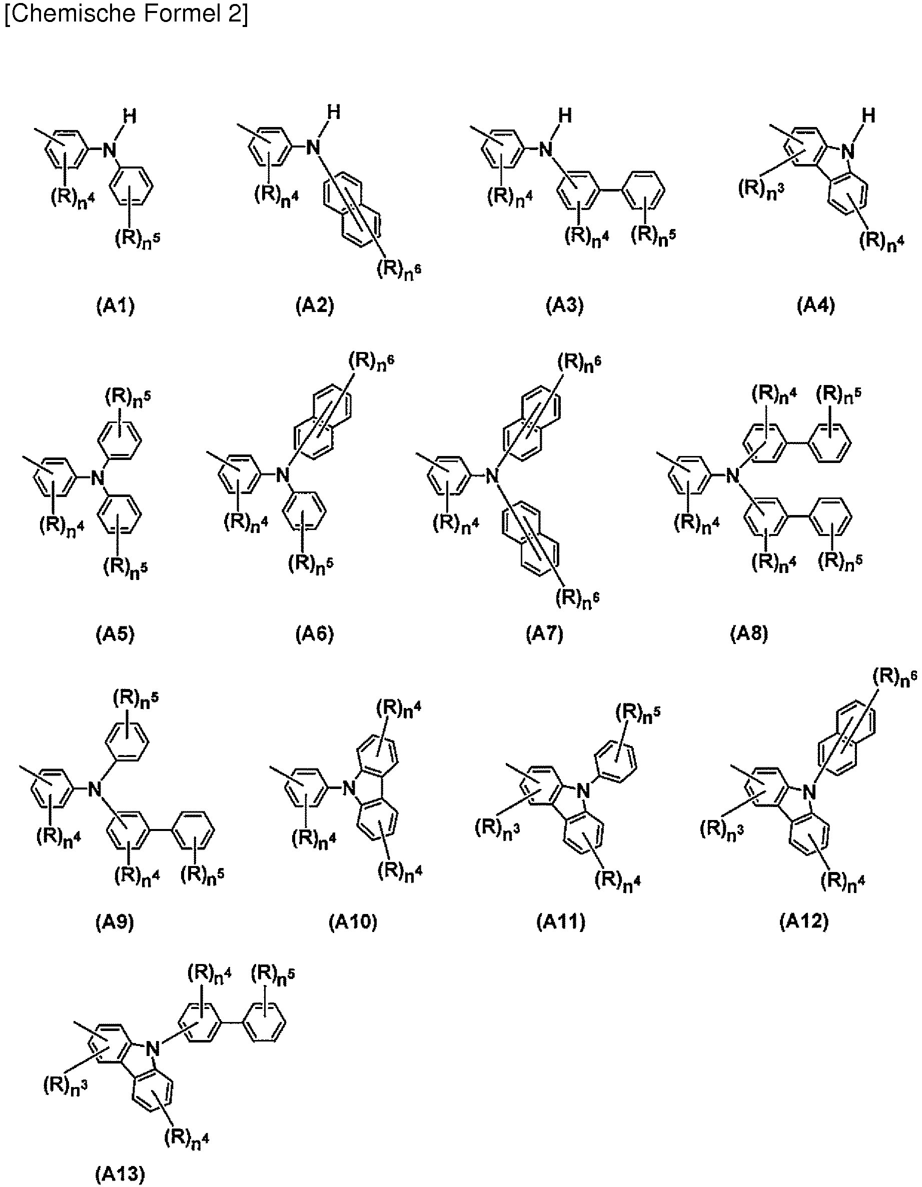 (worin R ein Halogenatom, eine Nitrogruppe, eine Cyanogruppe, eine gegebenenfalls mit Z³ substituierte Alkylgruppe mit 1 bis 20 Kohlenstoffatomen, eine gegebenenfalls mit Z³ substituierte Alkenylgruppe mit 2 bis 20 Kohlenstoffatomen, eine gegebenenfalls mit Z³ substituierte Alkinylgruppe mit 2 bis 20 Kohlenstoffatomen, eine gegebenenfalls mit Z¹ substituierte Alkoxygruppe mit 1 bis 20 Kohlenstoffatomen, eine gegebenenfalls mit Z³ substituierte Alkenyloxygruppe mit 2 bis 20 Kohlenstoffatomen oder eine gegebenenfalls mit Z³ substituierte Alkinyloxygruppe mit 2 bis 20 Kohlenstoffatomen, wobei die jeweiligen Gruppen R gleich oder unterschiedlich sind; und
n³ bis n⁶ für die Anzahl an Substituenten R stehen, wobei n³ eine ganze Zahl von 0 bis 3 ist, n⁴ eine ganze Zahl von 0 bis 4 ist, n⁵ eine ganze Zahl von 0 bis 5 ist und n⁶ eine ganze Zahl von 0 bis 7 ist, wobei n³ bis n⁶ jeweils gleich oder unterschiedlich sind;
wobei die Alkylgruppe, Alkenylgruppe, Alkinylgruppe, Alkyloxygruppe, Alkenyloxygruppe und Alkinyloxygruppe unverzweigt, verzweigt oder zyklisch sein können.)

2. Fluorenderivat nach Anspruch 1, worin R¹ und R² beide eine Alkylgruppe mit 2 bis 20 Kohlenstoffatomen sind, die zumindest eine Etherstruktur umfasst.

3. Fluorenderivat nach Anspruch 1 oder 2, worin n¹ und n² beide 0 sind.

4. Ladungstransportierende Substanz, bestehend aus einem Fluorenderivat nach einem der Ansprüche 1 bis 3.

5. Ladungstransportierender Anstrich, umfassend eine ladungstransportierende Substanz nach Anspruch 4 und ein organisches Lösungsmittel.

6. Ladungstransportierender Anstrich nach Anspruch 5, der weiters eine Dotierungssubstanz umfasst.

7. Ladungstransportierender Dünnfilm, hergestellt unter Verwendung eines ladungstransportierenden Anstrichs nach Anspruch 5 oder 6.

8. Organische elektrolumineszierende Vorrichtung, umfassend einen ladungstransportierenden Dünnfilm nach Anspruch 7.

9. Verfahren zur Herstellung eines Fluorenderivats nach Anspruch 1, wobei das Verfahren **dadurch gekennzeichnet ist, dass** es den Schritt des Ausführens einer Kreuzkupplungsreaktion zwischen einer Borsäureesterverbindung der Formel (1") oder (1''') und Verbindungen der Formel (A') und (A") in Gegenwart eines Katalysators umfasst: 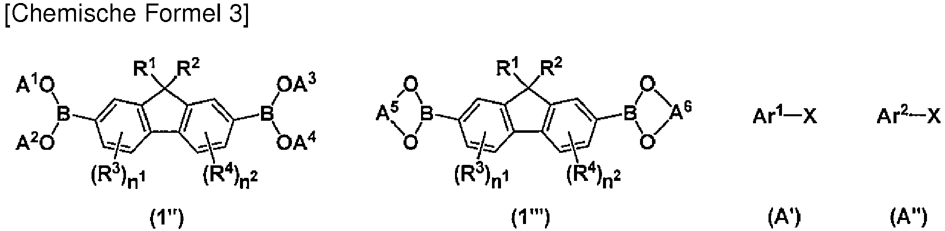 (worin R¹ bis R⁴, Ar¹, Ar², n¹ und n² wie in Anspruch 1 definiert sind; die X jeweils unabhängig voneinander ein Halogenatom oder eine Pseudohalogengruppe sind; A¹ bis A⁴ jeweils unabhängig voneinander ein Wasserstoffatom, eine Alkylgruppe mit 1 bis 20 Kohlenstoffatomen oder eine Arylgruppe mit 6 bis 20 Kohlenstoffatomen sind; und A⁵ und A⁶ jeweils unabhängig voneinander eine Alkandiylgruppe mit 1 bis 20 Kohlenstoffatomen oder eine Arylengruppe mit 6 bis 20 Kohlenstoffatomen sind).

## Revendications

1. Dérivé de fluorène **caractérisé en ce qu'**il répond à la formule (1) 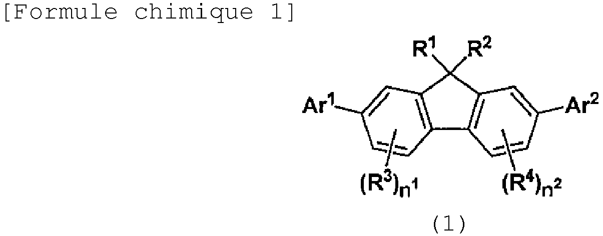
dans laquelle chacun de R¹ et R² est indépendamment un atome d'hydrogène, un groupe alkyle de 1 à 20 atomes de carbone, un groupe alcényle de 2 à 20 atomes de carbone, un groupe alcynyle de 2 à 20 atomes de carbone, un groupe aryle de 6 à 20 atomes de carbone, un groupe hétéroaryle de 2 à 20 atomes de carbone, un groupe alcoxy de 1 à 20 atomes de carbone, un groupe alcényloxy de 2 à 20 atomes de carbone, un groupe alcynyloxy de 2 à 20 atomes de carbone, un groupe aryloxy de 6 à 20 atomes de carbone, un groupe hétéroaryloxy de 2 à 20 atomes de carbone, ou un groupe alkyle de 2 à 20 atomes de carbone ayant au moins une structure d'éther (sous réserve qu'au moins l'un parmi R¹ et R² soit ledit groupe alcoxy, groupe alcényloxy, groupe alcynyloxy, groupe aryloxy, groupe hétéroaryloxy ou groupe alkyle ayant au moins une structure d'éther) ;
chacun de R³ et R⁴ est indépendamment un atome d'halogène, un groupe nitro, un groupe cyano, un groupe alkyle de 1 à 20 atomes de carbone qui peut être substitué par Z¹, un groupe alcényle de 2 à 20 atomes de carbone qui peut être substitué par Z¹, un groupe alcynyle de 2 à 20 atomes de carbone qui peut être substitué par Z¹, un groupe alcoxy de 1 à 20 atomes de carbone qui peut être substitué par Z¹, un groupe alcényloxy de 2 à 20 atomes de carbone qui peut être substitué par Z¹, un groupe alcynyloxy de 2 à 20 atomes de carbone qui peut être substitué par Z¹, un groupe aryle de 6 à 20 atomes de carbone qui peut être substitué par Z², un groupe hétéroaryle de 2 à 20 atomes de carbone qui peut être substitué par Z², un groupe aryloxy de 6 à 20 atomes de carbone qui peut être substitué par Z², ou un groupe hétéroaryloxy de 2 à 20 atomes de carbone qui peut être substitué par Z², les groupes R³ respectifs et les groupes R⁴ respectifs étant mutuellement identiques ou différents ;
Z¹ est un atome d'halogène, un groupe nitro, un groupe cyano, un groupe aryle de 6 à 20 atomes de carbone qui peut être substitué par Z³, un groupe hétéroaryle de 2 à 20 atomes de carbone qui peut être substitué par Z³, un groupe alcoxy de 1 à 20 atomes de carbone qui peut être substitué par Z³, un groupe alcényloxy de 2 à 20 atomes de carbone qui peut être substitué par Z³, un groupe alcynyloxy de 2 à 20 atomes de carbone qui peut être substitué par Z³, un groupe aryle de 6 à 20 atomes de carbone qui peut être substitué par Z³, ou un groupe hétéroaryle de 2 à 20 atomes de carbone qui peut être substitué par Z³ ;
Z² est un atome d'halogène, un groupe nitro, un groupe cyano, un groupe alkyle de 1 à 20 atomes de carbone qui peut être substitué par Z³, un groupe alcényle de 2 à 20 atomes de carbone qui peut être substitué par Z³, un groupe alcynyle de 2 à 20 atomes de carbone qui peut être substitué par Z³, un groupe alcoxy de 1 à 20 atomes de carbone qui peut être substitué par Z³, un groupe alcényloxy de 2 à 20 atomes de carbone qui peut être substitué par Z³, un groupe alcynyloxy de 2 à 20 atomes de carbone qui peut être substitué par Z³, un groupe aryle de 6 à 20 atomes de carbone qui peut être substitué par Z³, ou un groupe hétéroaryle de 2 à 20 atomes de carbone qui peut être substitué par Z³ ;
Z³ est un atome d'halogène, un groupe nitro ou un groupe cyano,
n¹ et n² représentent les nombres respectivement de substituants R³ et R⁴, et chacun est indépendamment un entier de 0 à 3 ; et
chacun d'Ar¹ et Ar² est indépendamment un groupe répondant à l'une quelconque des formules (A1) à (A13) 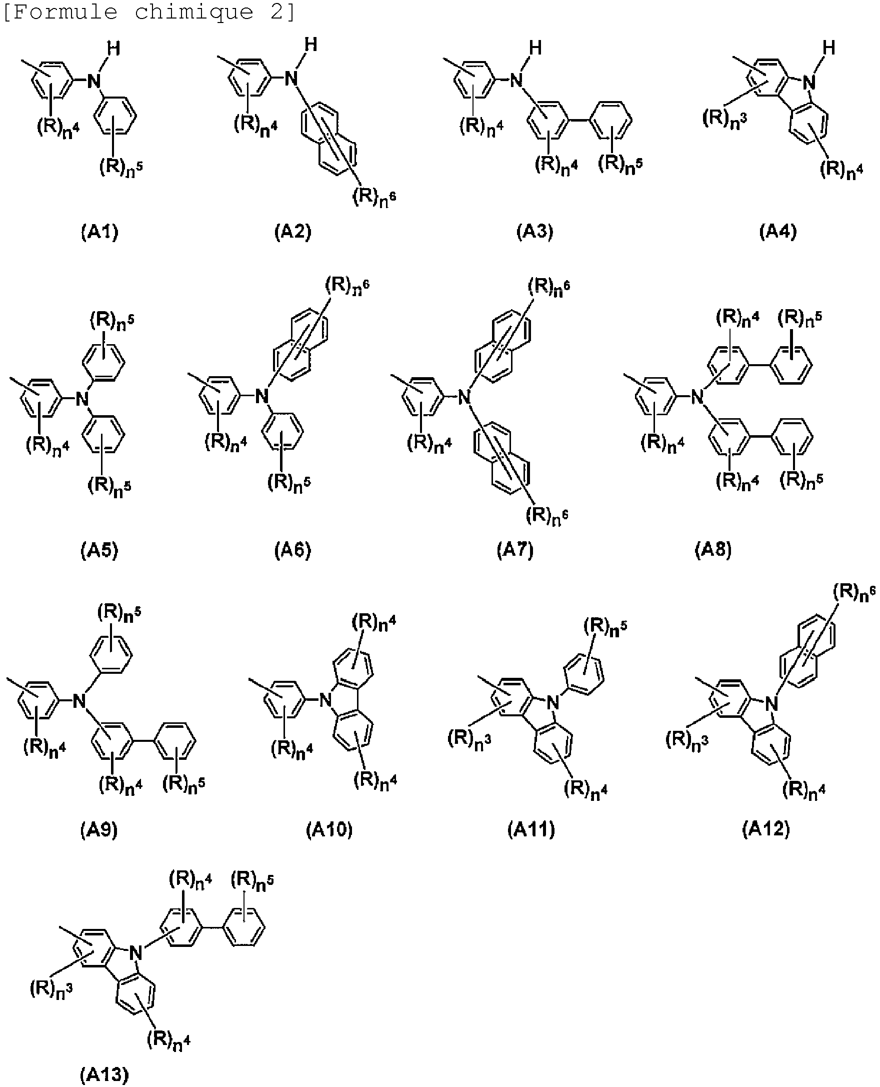 (où R est un atome d'halogène, un groupe nitro, un groupe cyano, un groupe alkyle de 1 à 20 atomes de carbone qui peut être substitué par Z³, un groupe alcényle de 2 à 20 atomes de carbone qui peut être substitué par Z³, un groupe alcynyle de 2 à 20 atomes de carbone qui peut être substitué par Z³, un groupe alcoxy de 1 à 20 atomes de carbone qui peut être substitué par Z³, un groupe alcényloxy de 2 à 20 atomes de carbone qui peut être substitué par Z³, ou un groupe alcynyloxy de 2 à 20 atomes de carbone qui peut être substitué par Z³, les groupes R respectifs étant identiques ou différents ; et
n³ à n⁶ représentent les nombres de substituants R, n³ étant un entier de 0 à 3, n⁴ étant un entier de 0 à 4, n⁵ étant un entier de 0 à 5 et n⁶ étant un entier de 0 à 7, n³ à n⁶ étant identiques ou différents ;
et dans laquelle le groupe alkyle, le groupe alcényle, le groupe alcynyle, le groupe alkyloxy, le groupe alcényloxy et le groupe alcynyloxy peuvent être linéaires, ramifiés ou cycliques.

2. Dérivé de fluorène selon la revendication 1, dans lequel R¹ et R² sont tous deux des groupes alkyle de 2 à 20 atomes de carbone qui contiennent au moins une structure d'éther.

3. Dérivé de fluorène selon la revendication 1 ou 2, dans lequel n¹ et n² valent tous deux 0.

4. Substance de transport de charges constituée du dérivé de fluorène de l'une quelconque des revendications 1 à 3.

5. Vernis de transport de charges comprenant la substance de transport de charges de la revendication 4 et un solvant organique.

6. Vernis de transport de charges selon la revendication 5, qui comprend en outre une substance dopante.

7. Film mince de transport de charges produit par utilisation du vernis de transport de charges de la revendication 5 ou 6.

8. Dispositif électroluminescent organique comprenant le film mince de transport de charges de la revendication 7.

9. Procédé de préparation du dérivé de fluorène selon la revendication 1, lequel procédé est **caractérisé en ce qu'**il comprend l'étape de mise en oeuvre d'une réaction de couplage croisé entre un composé ester d'acide borique de formule (1") ou (1"') et des composés de formules (A') et (A") en présence d'un catalyseur 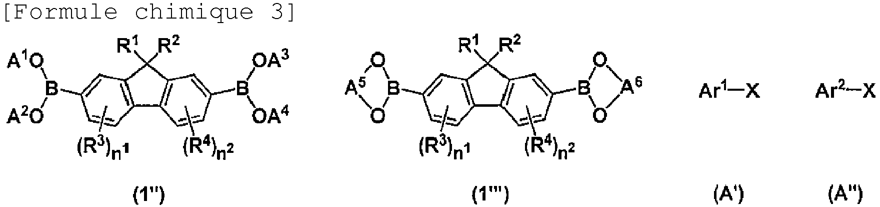 (où R¹ à R⁴, Ar¹, Ar², n¹ et n² sont tels que définis dans la revendication 1 ; chaque X est indépendamment un atome d'halogène ou un groupe pseudo-halogéno ; chacun d'A¹ à A⁴ est indépendamment un atome d'hydrogène, un groupe alkyle de 1 à 20 atomes de carbone ou un groupe aryle de 6 à 20 atomes de carbone ; et chacun d'A⁵ et A⁶ est indépendamment un groupe alcanediyle de 1 à 20 atomes de carbone ou un groupe arylène de 6 à 20 atomes de carbone).
